(19) Europäisches Patentamt
European Patent Office
Office européen des brevets

(11) EP 4 442 859 A1

# (12) EUROPÄISCHE PATENTANMELDUNG

(43) Veröffentlichungstag:
**09.10.2024 Patentblatt 2024/41**

(21) Anmeldenummer: **23166923.5**

(22) Anmeldetag: **06.04.2023**

(51) Internationale Patentklassifikation (IPC):
***C25B 1/04*** *(2021.01)* ***C25B 1/23*** *(2021.01)*
***C25B 1/26*** *(2006.01)* ***C25B 3/09*** *(2021.01)*
***C25B 3/29*** *(2021.01)* ***C25B 15/08*** *(2006.01)*
***C01B 3/12*** *(2006.01)* ***C01B 32/40*** *(2017.01)*
***C01B 32/80*** *(2017.01)* ***C01C 1/04*** *(2006.01)*
***C07C 1/20*** *(2006.01)* ***C07C 29/151*** *(2006.01)*
***C07C 209/48*** *(2006.01)* ***C07C 253/26*** *(2006.01)*
***C07C 263/10*** *(2006.01)* ***C08G 18/73*** *(2006.01)*

(52) Gemeinsame Patentklassifikation (CPC):
(C-Sets verfügbar)
**C25B 1/04; C01B 3/12; C01B 32/40; C01B 32/80; C01C 1/0405; C07C 1/20; C07C 29/1518; C07C 41/09; C07C 209/48; C07C 253/26; C07C 253/30; C07C 263/10; C08G 18/42; C08G 18/48; C08G 18/73;** (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Covestro Deutschland AG**
**51373 Leverkusen (DE)**

(72) Erfinder:
- **Bulan, Andreas**
  **40764 Langenfeld (DE)**
- **Weber, Rainer**
  **51519 Odenthal (DE)**

(74) Vertreter: **Levpat**
**c/o Covestro AG**
**Gebäude K12**
**51365 Leverkusen (DE)**

## (54) NACHHALTIGE HERSTELLUNG VON HEXAMETHYLENDIISOCYANAT FÜR DIE PRODUKTION VON POLYURETHAN

(57) Die vorliegende Erfindung betrifft ein Herstellungsverfahren für Hexamethylendiisocyanat nach Anspruch 1, sowie für die Herstellung von Polyurethan aus Hexamethylendiisocyanat, wobei in dem jeweiligen Verfahren Methanol als Rohstoff für den Aufbau der Hexamethylen-Einheit des Hexamethylendiisocyanates eingesetzt wird.

EP 4 442 859 A1

(52) Gemeinsame Patentklassifikation (CPC): (Forts.)
**C25B 1/23; C25B 1/26; C25B 3/09; C25B 3/295;**
**C25B 15/081;** C01B 2203/0415; C01B 2203/046;
C01B 2203/0475; C07C 2529/40

C-Sets
**C07C 1/20, C07C 11/06;**
**C07C 29/1518, C07C 31/04;**
**C07C 41/09, C07C 43/043;**
**C07C 209/48, C07C 211/12;**
**C07C 253/26, C07C 255/08;**
**C07C 253/30, C07C 255/04;**
**C07C 263/10, C07C 265/14**

## Beschreibung

Die vorliegende Erfindung betrifft ein Herstellungsverfahren von Hexamethylendiisocyanat für die Synthese von Polyurethan, sowie ein Verfahren zur Herstellung von Polyurethan aus dem gemäß vorgenanntem Herstellungsverfahren erhaltenen Hexamethylendiisocyanat und ein Mehrkomponenten-System zur Hexamethylendiisocyanat Herstellung gemäß vorgenanntem Herstellungsverfahren.

Bisher basiert die industrielle Herstellung von Hexamethylendiisocyanat (auch als HDI, als Hexan-1,6-diyldiisocyanat oder als 1,6-Diisocyanatohexan bezeichnet) überwiegend auf der Nutzung fossiler Rohstoffe, wie z.B. von aus Erdgas bereitgestelltem Synthesegas und erdölbasierten aromatischen Verbindungen. Die konsequente Nutzung von regenerativen Rohstoffen und/oder von Wertstoffen aus Abfällen für die Herstellung von Materialien wie Hexamethylendiisocyanat ist ein Ziel für die Bereitstellung nachhaltiger Kunststoffe, wie Polyurethan und Materialien daraus.

Weiterhin wird die Nutzung von Energie aus regenerativen Energiequellen für die industrielle Herstellung chemischer Rohstoffe, wie z.B. von Hexamethylendiisocyanat, angestrebt.

Es war somit eine Aufgabe der vorliegenden Erfindung, ein nachhaltigeres Herstellungsverfahren für Hexamethylendiisocyanat im industriellen Maßstab bereitzustellen, welches die Nutzung regenerativer Rohstoffe ermöglicht. Zudem war es eine weitere Aufgabe der Erfindung, eine Methode zur Herstellung von Hexamethylendiisocyanat bereitzustellen, mit deren Hilfe trotz möglicher Schwankungen in der Verfügbarkeit regenerativer Energie ein emissionsarmes Hexamethylendiisocyanat für die Herstellung von Polyurethanen erreicht werden kann. Unter "regenerativer Energie" versteht der Fachmann Energie aus einer Energiequelle, die sich nicht erschöpft, wie z.B. Windkraft, Wasserkraft, Bioenergie (z.B. Verstromung von Biogas oder Biomasse) oder Sonnenenergie.

Unter "Nachhaltigkeit" eines Verfahrens versteht der Fachmann in Anwendung der durch die UN geprägte Nachhaltigkeits-Definition ("sustainable development") gemäß Brundtlandbericht der "Weltkommission für Umwelt und Entwicklung", dass durch die Ausführung des Verfahrens in der Gegenwart ein möglichst geringer bis gar kein Beitrag geleistet wird, dass künftige Generationen der Menschheit ihre eigenen Bedürfnisse nicht mehr befriedigen können, insbesondere Bedürfnisse mit Blick auf die Nutzung von Ressourcen wie z.B. fossiler Rohstoffe und insbesondere mit Blick auf die Schonung des Lebensraumes, wie z.B. den Schutz der Erdatmosphäre. Die Erfindung hat somit zur Aufgabe, die Produktion von Hexamethylendiisocyanat, sowie daraus hergestelltem Polyurethan nachhaltiger als die aus dem Stand der Technik bekannten Produktionsmethoden zu gestalten. Der Beitrag der Produktion von Hexamethylendiisocyanat und Polyurethan zu einer sinkenden Befriedigung der Bedürfnisse zukünftiger Generationen soll vermindert bis vermieden werden.

Ein erster Gegenstand der Erfindung ist daher ein Verfahren zur Herstellung von Hexamethylendiisocyanat für die Herstellung von Polyurethan, enthaltend zumindest die Schritte:

a) Bereitstellung von Hexamethylendiamin, welches ein Verfahrensprodukt eines Verfahrens enthaltend mindestens folgende Schritte ist:

i) Bereitstellung von Methanol, welches ein Verfahrensprodukt eines Verfahrens mit mindestens folgenden Schritten ist:

i-1) Bereitstellung von Kohlenmonoxid als Verfahrensprodukt mindestens einer partiellen Reduktion von $CO_2$ zu CO und/oder als Verfahrensprodukt mindestens einer partiellen Oxidation von organischem Material zu CO ist, wobei mindestens ein organisches Material ausgewählt wird aus mindestens einer organischen, festförmigen Verbindung, Methan aus biologischer Quelle oder Mischungen daraus;

i-2) Umsetzung des bereitgestellten Kohlenmonoxids zu Methanol;

ii) Synthese von Propen aus zuvor bereitgestelltem Methanol durch zumindest folgende Schritte:

ii-1) Umsetzung des zuvor bereitgestellten Methanols zu einer Produktmischung, enthaltend Dimethylether, Wasser und Methanol;

ii-2) Umwandlung von Ausgangsmaterial, enthaltend Dimethylether, Wasser und Methanol jeweils aus der vorgenannten Produktmischung, bei einer Temperatur von mehr als 200°C durch Kontakt mit einem Katalysator, vorzugsweise mit mindestens einer Zeolith-Verbindung als Katalysator, zu Propen;

iii) Synthese von Acrylnitril aus dem Propen durch zumindest folgende Schritte:

iii-1) Bereitstellung von Ammoniak, welcher ein Verfahrensprodukt eines Verfahrens mit mindestens folgenden Schritten ist:

iii-1-1) Bereitstellung von Wasserstoffgas als Produkt einer Elektrolyse von Wasser, bevorzugt unter Einsatz von aus regenerativer Energie erzeugter

elektrischer Energie;

iii-1-2) Umsetzung des bereitgestellten Wasserstoffgases mit gasförmigem Stickstoff zu Ammoniak;

iii-2) Umsetzung des Propens mit dem bereitgestellten Ammoniak zu Acrylnitril;

iv) Synthese von Adiponitril aus dem Acrylnitril durch zumindest folgende Schritte:

Einbringung einer Mischung, enthaltend Wasser, besagtes Acrylnitril und mindestens ein Elektrolytsalz in den Kathodenraum einer Elektrolysezelle und Inkontaktbringen der Mischung mit einer Kathode, an der elektrischer Strom anliegt;

kathodische Hydrodimerisierung des Acrylnitrils unter Bildung einer Adiponitril-haltigen Produktmischung;

Ausbringung der Adiponitril-haltigen Produktmischung aus dem Kathodenraum der Elektrolysezelle und optional Aufreinigung des Adiponitrils;

v) Synthese von Hexamethylendiamin aus dem Adiponitril durch zumindest folgende Schritte:

Bereitstellung von Wasserstoffgas als Produkt einer Elektrolyse, bevorzugt unter Einsatz von aus regenerativer Energie erzeugter elektrischer Energie;

Hydrierung des Adiponitrils mit dem Wasserstoffgas zu Hexamethylendiamin;

b) Herstellung von Phosgen durch zumindest folgende Verfahrensschritte:

i) Bereitstellung von Kohlenmonoxid als Verfahrensprodukt mindestens einer partiellen Reduktion von $CO_2$ zu CO und/oder als Verfahrensprodukt mindestens einer partiellen Oxidation von organischem Material zu CO, wobei mindestens ein organisches Material ausgewählt wird aus mindestens einer organischen, festförmigen Verbindung, einer organischen, flüssigen Monohydroxyalkylverbindung, Methan aus biologischer Quelle oder Mischungen daraus;

ii) Umsetzung des Kohlenmonoxids aus Schritt i) mit Chlor zu Phosgen;

c) Umsetzung des bereitgestellten Hexamethylendiamins mit dem Phosgen zu Hexamethylendiisocyanat.

Eine "katalysierte Reaktion" oder "katalytische Umsetzung" findet erfindungsgemäß unter Einsatz eines Katalysators statt, der die Produktbildung aus mindestens einem Reaktanden (z.B. Kohlendioxid oder Methanol) im Vergleich zur gleichen Reaktion unter gleichen Reaktionsbedingungen aber in Abwesenheit des Katalysators durch Herabsetzung der aufzuwendenden Energie und/oder durch eine Selektivitätssteigerung unter Erhöhung der Produktausbeute katalysiert. Beispielsweise ist die Umwandlung gemäß Schritt a) ii-2) eine katalytische Umsetzung des besagten Ausgangsmaterials zu Propen.

Gemäß vorliegender Erfindung ist ein Material oder eine chemische Verbindung organisch, wenn das Material / die chemische Verbindung mindestens eine kovalente Kohlenstoff-Wasserstoff-Bindung enthält.

Als festförmig ist ein Stoff bzw. ein Material definiert, wenn der Stoff bei 25°C und 1013 mbar als Feststoff vorliegt. Als flüssig ist ein Stoff bzw. ein Material definiert, wenn der Stoff bei 25°C und 1013 mbar als Flüssigkeit vorliegt.

In Schritt a) des erfindungsgemäßen Verfahrens wird Hexamethylendiamin (auch als 1,6-Diaminohexan oder als Hexan-1,6-diamin bezeichnet) bereitgestellt. Für die Bereitstellung des Hexamethylendiamins ist es erfindungsgemäß ausreichend, wenn das bereitgestellte Hexamethylendiamin ein tatsächliches Verfahrensprodukt mindestens der unter a) genannten Schritte i), ii), iii), iv) und v) ist. Dies bedeutet, dass es für die Ausführung des Schrittes der Bereitstellung des Hexamethylendiamin bereits ausreicht, das auf besagte Weise hergestellte Hexamethylendiamin lediglich im Rahmen einer Belieferung als Rohstoff aus einem Lagerbehälter oder aus einer Zuleitung zu entnehmen, um dieses zumindest dem Schritt c) des erfindungsgemäßen Verfahrens zuzuführen. In diesem Falle führt der Hexamethylendiamin-Produzent als Ausführender des erfindungsgemäßen Verfahrens die unter a) des erfindungsgemäßen Verfahrens genannten Schritte i) und ii) zur Herstellung des Hexamethylendiamins nicht selbst aus, sondern er sorgt nur dafür, dass das bereitgestellte Hexamethylendiamin durch Anwendung mindestens der unter a) genannten Schritte i) und ii) hergestellt wurde und somit deren tatsächliches Verfahrensprodukt ist.

Ebenso ist es erfindungsgemäß möglich, wenn zur Bereitstellung des Hexamethylendiamins die mindestens unter a) genannten Schritte i) bis v) zur Herstellung des Hexamethylendiamins als integrale Schritte eines erfindungsgemäßen Verfahrens zur Herstellung von Hexamethylendiisocyanats durch den Hexamethylendiisocyanat-Produzenten ausgeführt werden und dabei erhaltenes Hexamethylendiamin, gegebenenfalls nach einer Zwischenlagerung in einem Lagerbehälter, zumindest dem Schritt c) des erfindungsgemäßen Verfahrens zugeführt wird.

Die Möglichkeiten der Bereitstellung, i.e. Belieferung oder eigene Herstellung des speziellen Hexamethylendiamins durch den Hexamethylendiisocyanat-Produzenten, gelten jeweils auch für die nachfolgend genannten Ausführungsformen der unter a) genannten Schritte i) bis v).

Der im erfindungsgemäßen Verfahren unter a) genannte Schritt i) erfordert die Bereitstellung von Methanol, welches ein tatsächliches Verfahrensprodukt eines Verfahrens mit mindestens folgenden Schritten ist: i-1) Umsetzung von CO zu Methanol, wobei das CO ein Verfahrensprodukt mindestens einer partiellen Reduktion von $CO_2$ zu CO und/oder ein Verfahrensprodukt mindestens einer partiellen Oxidation von organischem Material zu CO ist, wobei mindestens ein organisches Material ausgewählt wird aus mindestens einer organischen, festförmigen Verbindung, Methan aus biologischer Quelle oder Mischungen daraus.

Für die Bereitstellung des Methanols ist es gemäß vorliegender Erfindung ausreichend, wenn das Methanol ein tatsächliches Verfahrensprodukt mindestens der unter i-1) genannten Umsetzung ist, wobei für diese Umsetzung Kohlenmonoxid (Kohlenmonoxid auch als CO bezeichnet) genutzt wird, welches wiederum ein tatsächliches Verfahrensprodukt eines speziellen Verfahrens ist. Dies bedeutet, dass es für die Ausführung des Schrittes der Bereitstellung des Methanols bereits ausreicht, das auf besagte Weise hergestellte Methanol lediglich im Rahmen einer Belieferung als Rohstoff aus einem Lagerbehälter oder aus einer Zuleitung zu entnehmen. In diesem Falle führt der Hexamethylendiisocyanat-Produzent als Ausführender des erfindungsgemäßen Verfahrens oder der Lieferant des für das erfindungsgemäße Verfahren bereitgestellte Hexamethylendiamin die vorgenannte Umsetzung zur Herstellung des Methanols nicht selbst aus, sondern er sorgt nur dafür, dass das für die Bereitstellung des Hexamethylendiamin bereitgestellte Methanol durch Anwendung mindestens des besagten Schritts i-1) hergestellt wurde und somit deren tatsächliches Verfahrensprodukt ist.

Ebenso ist es erfindungsgemäß möglich, wenn zur Bereitstellung des Methanols mindestens vorgenannte Umsetzung zur Herstellung des Methanols als integraler Schritt des erfindungsgemäßen Verfahrens zur Herstellung von Hexamethylendiisocyanat durch den Hexamethylendiisocyanat-Produzenten oder den Lieferanten des bereitgestellten Hexamethylendiamins ausgeführt werden und das dabei erhaltene Methanol, gegebenenfalls nach einer Zwischenlagerung in einem Lagerbehälter, der unter Schritt a) des Verfahrens vorgesehenen Synthese von Hexamethylendiamin unter Ausführung zumindest der Schritte ii-1), ii-2), iii), iv) und v) zugeführt wird.

Die Möglichkeiten der Bereitstellung, i.e. Belieferung oder eigene Herstellung des speziellen Methanols durch den Hexamethylendiisocyanat-Produzenten, gelten jeweils auch für die nachfolgend genannten Ausführungsformen der Bereitstellung des Methanols.

Für die Herstellung von Methanol werden üblicherweise Kohlenstoffoxide zusammen mit Wasserstoff (das Gemisch auch als Synthesegas bezeichnet) umgesetzt. Dieser Prozess ist exotherm und kann durch folgende Reaktionsgleichungen beschrieben werden:

$$CO + 2H_2 \rightleftharpoons CH_3OH$$

$$CO_2 + 3H_2 \rightleftharpoons CH_3OH + H_2O$$

Beide Reaktionen werden durch die ebenfalls exotherme Water-Gas-Shift Reaktion gekoppelt, welche wie folgt beschrieben werden kann:

$$CO + H_2O \rightleftharpoons CO_2 + H_2$$

Herkömmlicherweise wird für die Methanolsynthese hauptsächlich aus CO und $H_2$ bestehendes Synthesegas eingesetzt, welches wiederum aus Erdgas (fossiles Methan) und Wasserdampf in einem Reformerprozess (steam reforming) hergestellt wird. Die Zusammensetzung des Synthesegases hat starken Einfluss auf die optimale Nutzung. Die Zusammensetzung kann über die Stöchiometriezahl (SZ) beschrieben werden:

$$SZ = \frac{n_{H_2} - n_{CO_2}}{n_{CO} + n_{CO_2}}$$

Für SZ = 2 liegen die Reaktanden im stöchiometrischen Verhältnis gemäß der o.g. Reaktionsgleichungen vor. Real werden jedoch leicht höhere Werte für SZ (2,01-2,1) verwendet, wobei dies durch einen höheren Wasserstoffanteil im Synthesegas realisiert wird (Dittmeyer et al. "Chemische Technik", Band 4, 5. Auflage).

In dem erfindungsgemäßen Verfahren wird das für die Umsetzung zur Methanolbereitstellung benötigte Kohlenmonoxid eben nicht durch die das herkömmliche steam reforming unter Einsatz fossiler Kohlenstoffquellen (Erdgas) bereitgestellt, sondern durch partielle Reduktion von Kohlendioxid und/oder durch partielle Oxidation (und nötigenfalls Gasifizierung) von besagtem organischem Material zu CO.

Bei der partiellen Oxidation von organischem, festförmigem Material (bevorzugt von organischem, polymeren Material) erfolgt gemäß dem erfindungsgemäßen Verfahren die Bereitstellung von CO aus organischem, festförmigen Material (bevorzugt aus gebrauchten Polymer-Abfallfraktionen) und Sauerstoff-haltigem Gas durch Umwandlung des besagten organischen, festförmigen Materials unter partieller Oxidation in ein Gas, enthaltend CO (im Folgenden auch als Gasifizierung bezeichnet).

Durch den Einsatz von Polymer-haltigen Abfallfraktionen, die beispielsweise überwiegend aus PET- ,

PE-, PP-, Polyurethan- oder Polycarbonat-haltigen Abfallfraktionen bestehen können (nachfolgend Polymer-Abfallfraktion genannt), zur Bereitstellung von CO, werden diese Polymer-haltigen Abfallfraktionen einer Wiederverwertung zugefügt und dadurch Hexamethylendiisocyanat und auch Polyurethan aus diesem Hexamethylendiisocyanat mit verbesserter Nachhaltigkeit hergestellt.

Eine "polymere Verbindung" ist ein Molekül mit einer relativen Molmasse (Mw) von mindestens 2000 g/mol, dessen chemische Struktur überwiegend sich mehrfach wiederholende Struktureinheiten umfasst, die sich von einem oder mehreren verschiedenen Molekülen geringerer relativer Molmasse ableiten. Bei den im Rahmen dieser Anmeldung für Polymere bzw. polymere Verbindungen angegebenen mittleren Molmassen handelt es sich - sofern nicht explizit anders gekennzeichnet - stets um gewichtsmittlere Molmassen Mw, die grundsätzlich mittels Gelpermeationschromatographie mit Hilfe eines RI-Detektors bestimmbar sind, wobei die Messung zweckmäßig gegen einen externen Standard erfolgt. Ein "polymeres Material" ist ein Material, enthaltend mindestens eine polymere Verbindung.

Durch die partielle Oxidation werden organisches Material (bevorzugt organisches, festförmiges Material, besonders bevorzugt polymeres, organisches Material, ganz besonders bevorzugt die Polymer-Abfallfraktion) und Sauerstoff zur partiellen Oxidationsreaktion gebracht und zu einem Produktgasgemisch enthaltend Wasserstoff und CO und gegebenenfalls Nebenprodukte umgesetzt. Als Nebenprodukte gelten insbesondere Kohlenwasserstoffe mit 1 bis 8 Kohlenstoffatomen. Im Produktgasgemisch sind u.a. ebenso $CO_2$ und Wasserdampf vorhanden.

Als weiteres Nebenprodukt wird aus dem Gasifizierungs-Prozess eine nicht weiter umsetzbare Restfraktion erhalten.

Die für die Gasifizierung benötigte Temperatur wird zumindest teilweise durch partielle Verbrennung (partielle Oxidation) des organischen, festförmigen Materials (bevorzugt des polymeren, organischen Materials, besonders bevorzugt der Polymer-Abfallfraktion) mit einem sauerstoffhaltigen Gas erreicht. Die partielle Oxidation des eingebrachten organischen, festförmigen Materials wird in dem Reaktor bei einer Temperatur von mindestens 400°C durchgeführt. Vorzugsweise wird die partielle Oxidation des Materials in dem Reaktor bei einer Temperatur in einem Temperaturbereich von 600 °C bis 1500°C, insbesondere von 850°C bis 1400°C, weiter bevorzugt von 1 100°C bis 1300°C, durchgeführt.

Das als Verfahrensprodukt einer partiellen Oxidation bereitgestellte Kohlenmonoxid kann beispielsweise nach einem Verfahren zur Herstellung von Kohlenmonoxid für die Bereitstellung von Methanol hergestellt worden sein, enthaltend mindestens folgende Schritte

1) Bereitstellung eines sauerstoffhaltigen Gasstroms, enthaltend mindestens 50 Gew.-% Sauerstoffgas,

2) partielle Oxidation von organischem Material (bevorzugt von organischem Material, enthaltend mindestens eine polymere, organische Verbindung), wobei besagtes organisches Material in einen Reaktor einer Vorrichtung eingebracht und dort zumindest durch Zufuhr des besagten sauerstoffhaltigen Gasstromes und von Wärme bei mindestens 400°C unter Bildung eines Produktgases behandelt und das resultierende Produktgas, gegebenenfalls nach mindestens einem weiteren partiellen Oxidationsschritt des Produktgases, als ein Kohlenmonoxid-haltiger Produktgas-Strom gemeinsam mit darin dispergiertem, partikelförmigem Feststoff aus der Vorrichtung ausgebracht wird;

3) der Kohlenmonoxid-haltige Produktgas-Strom einer Reinigung zugeführt wird, in der zumindest

3-1) der Kohlenmonoxid-haltige Produktgas-Strom zur Abtrennung von Feststoff einem Waschschritt zugeführt wird, worin

(i) Wasser mit dem Kohlenmonoxid-haltigen Produktgas-Strom in Kontakt gebracht wird, wodurch der im Kohlenmonoxid-haltigen Produktgas-Strom dispergierte, partikelförmige Feststoff eine Schlacke bildet und diese Schlacke entfernt und abgeführt wird,

(ii) der von partikelförmigem Feststoff gereinigte, Kohlenmonoxid-haltige Produktgas-Strom ausgebracht wird;

3-2) ein mittels Waschschritt von partikelförmigem Feststoff gereinigter Kohlenmonoxid-haltiger Produktgas-Strom in einem Trockenschritt zumindest einer Abtrennung von Wasser zugeführt, Wasser abgetrennt und der resultierende Kohlenmonoxid-haltige Produktgas-Strom ausgebracht wird,

3-3) ein mittels Abtrennung von Wasser gereinigter Kohlenmonoxid-haltiger Produktgas-Strom zumindest einer Abtrennung von Kohlendioxid zugeführt, Kohlendioxid abgetrennt und der resultierende Kohlenmonoxid-haltige Produktgas-Strom und Kohlendioxid ausgebracht wird;

3-4) ein mittels Abtrennung von Kohlendioxid gereinigter Kohlenmonoxid-haltiger Produktgas-Strom zumindest einer Trenneinheit zur Abtrennung von Kohlenmonoxid zugeführt, eine Abtrennung von Kohlenmonoxid durchgeführt und das resultierende Kohlenmonoxid und ein Wasserstoffgas-haltiges Restgas ausgebracht wird;

3-5) optional ein mittels besagter Trenneinheit abgetrenntes, Wasserstoffgas-haltiges Restgas einer Restgasbehandlung zugeführt, Wasserstoffgas abgetrennt und Wasserstoffgas und ein Endgas ausgebracht wird.

Ein Feststoff ist bekanntermaßen "partikelförmig", wenn er in Form eines körnigen Gemenges aus einer Vielzahl an losen, festförmigen Partikeln des besagten Stoffes vorliegt, das wiederum sogenannte Körner umfasst. Ein Korn ist eine Bezeichnung für die partikulären Bestandteile von Pulvern (Körner sind die losen, festförmigen Partikel), Stäuben (Körner sind die losen festförmigen Partikel), Granulaten (lose, festförmige Partikel sind Agglomerate aus mehreren Körnern) und anderen körnigen Gemengen.

Ein "Reaktor" ist ein Volumen, in dem eine chemischen Umwandlung, z.B. eine partielle Oxidation von polymerer, organischer Verbindung eines Materials, stattfindet. Dies kann für die partielle Oxidation beispielsweise das Volumen eines beheizten Gefäßes sein, in dem sich das Material befindet.

Das für die partielle Oxidation benötigte Sauerstoffgas kann z.B. einer Wasserelektrolyse oder einer Luftzerlegungsanlage entnommen werden. Im Rahmen einer bevorzugten Ausführungsform der partiellen Oxidation, wird zur Bereitstellung des sauerstoffhaltigen Gasstroms eine Elektrolyse von Wasser unter Erhalt von Sauerstoffgas und Wasserstoffgas durchgeführt und das Sauerstoffgas aus dieser Elektrolyse zur Bereitstellung des sauerstoffhaltigen Gasstromes genutzt.

Eine Wasserelektrolyse kann mit Anlagen nach dem Stand der Technik durchgeführt werden. Bekannt und kommerziell erhältlich sind technische Anlagen zur alkalischen Wasserelektrolyse als auch zur Polymerelektrolyt-basierten Elektrolyse, der so genannten PEM-Elektrolyse. Die Prinzipien der Wasserelektrolyse sind beispielhaft in Kapitel 6.3.4 in Volkmar M. Schmidt in "Elektrochemische Verfahrenstechnik" (2003 Wiley-VCH-Verlag; ISBN 3-527-29958-0) beschrieben.

Das in den Reaktor für die partielle Oxidation eingebrachte organische, festförmige Material enthält erfindungsgemäß bevorzugt mindestens eine polymere, organische Verbindung. Die partielle Oxidation sollte in dem Reaktor möglichst gleichmäßig und selektiv stattfinden. Eine Steigerung dieser Parameter kann erzielt werden, wenn im Rahmen einer bevorzugten Ausführungsform jeweils bezogen auf den Zeitpunkt vor der Einbringung das Gewichtsverhältnis des im sauerstoffhaltigen Gasstrom enthaltenen Sauerstoffgases zur polymeren, organischen Verbindung innerhalb eines Gewichtsverhältnisbereiches von 0,4 zu 1,0 bis 1,2 zu 1,0, bevorzugt von 0,6 zu 1,0 bis 0,9 zu 1,0 in den Reaktor eingebracht werden.

Die partielle Oxidation des eingebrachten organischen, festförmigen Materials wird im Rahmen einer bevorzugten Ausführungsform bei einem absoluten Druck von mehr als 1 bar, bevorzugt bei einem absoluten Druck in einem Bereich von 2 bis 80 bar, besonders bevorzugt bei einem absoluten Druck in einem Bereich von 2 bis 50 bar, durchgeführt.

Die partielle Oxidation des organischen, festförmigen Materials kann in mindestens einem der folgenden drei Reaktoren ausgeführt werden: Reaktor für Flugstromvergasung, Reaktor für Wirbelschichtvergasung und Reaktor für Festbettvergasung.

Für die Verwendung in einem Reaktor für Flugstromvergasung muss das organische, festförmige Material, auf eine Korngröße mit einem mittleren Teilchendurchmesser $X_{50,3}$ von < 0,1 mm (Staub) gemahlen werden. Die Zufuhr in den Reaktor erfolgt entweder pneumatisch oder als Aufschlämmung. Die größte Einschränkung dieser Art der partiellen Oxidation für die chemische Wiederverwertung von Abfall ist die Mahlbarkeit und Förderbarkeit von Ausgangsstoffen aus heterogenem Abfall. Eine thermische Behandlung von Biomasse (Torrefaktion) bei 200 - 300 °C unter $O_2$ Ausschluss wird zur Herstellung einer "Biokohle" mit ähnlicher Mahlbarkeit wie Steinkohle verwendet. In einer anderen Variante kann eine vorgeschaltete Pyrolyse zur Herstellung eines pumpbaren Pyrolyseöls verwendet werden. Das durch Abfall-Pyrolyse gewonnene Öl kann entweder direkt oder in Form einer mit dem festen Pyrolyserückstand (Pyrolysekoks) vermischten Aufschlämmung partiell oxidiert werden. Diese Prozesskonfiguration wurde von der Firma Noell (Noell-Konversionsverfahren zur Verwertung und Entsorgung von Abfällen, Jürgen Carl EF-Verl. für Energie- und Umwelttechnik, 1994. ISBN: 3924511829) entworfen.

Eine andere Variante stellt die Nutzung eines Reaktors für Wirbelschichtvergasung dar. Die Vergasung von organischem, festförmigen Material, insbesondere von Abfall, in Wirbelschichtreaktoren ist vielfach mit den Technologien EBARA (Showa Denko, Japan), ENERKEM (Enerkem, Edmonton, Kanada) und der Großdemonstration der Hochtemperatur-Winkler-Gasförderung (HTW) durch die Rheinbraun AG (heute RWE) von 1993 bis 1997 in Berrenrath, Deutschland, bekannt. Die Vorbehandlung zur Einspeisung des organischen Materials, bzw. des polymere, organische Verbindungen enthaltenden Materials, in den Reaktor erfordert eine Zerkleinerung auf mit einem mittleren Teilchendurchmesser $X_{50,3}$ von 30 - 80 mm. Die Einspeisung in den Reaktorbehälter erfolgt über Schneckenförderer begrenzt den Vergaserdruck auf maximal 10 bar. Die Technologien von ENERKEM und EBARA ermöglichen die Vergasung von hochkalorischem Abfall (Kunststoffabfall oder kunststoffreiche Ersatzbrennstoffe. Wirbelschichtvergaser werden bei milden Temperaturen von 700 - 950 °C deutlich unterhalb des Asche-Schmelzpunkts des Einsatzmaterials betrieben, um Anbackungen und Agglomerationen im Reaktor zu vermeiden. Ein weiterer Vorteil dieser milden Reaktortemperatur ist der unvollständige Kohlenstoffumsatz in der Wirbelschicht. Weiterhin enthält das Rohgas aus Reaktoren zur Wirbelschichtvergasern typischerweise erhebliche Mengen an Methan und anderen Kohlenwasserstoffen. Um dies zu kompensieren und eine hohe syn-Gasausbeute von $H_2$ und CO zu gewährleisten, nutzen die Prozesse von

ENERKEM und EBARA eine zweite Hochtemperatur-Stufe zur partiellen Oxidation (ca. 1400 °C), die direkt hinter dem Wirbelbett angeordnet ist, um Flugasche zu schmelzen und Kohlenwasserstoffe im Produktgas der ersten Stufe zum finalen Kohlenmonoxid-haltigen Produktgasstrom umzuwandeln. ENERKEM nennt diese zweite Stufe "Thermoreformer", EBARA bezeichnet einen "Hochtemperatur-Vergasungsofen". Die hohe Temperatur dieser zweiten partiellen Oxidationsstufe erhöht die $CO_2$-Produktion.

Das CO für die Bereitstellung des Methanols kann ebenso bevorzugt als Verfahrensprodukt aus der Umwandlung von Methan aus biologischer Quelle mittels partieller Oxidation in einem klassischen Reformerprozess stammen. Im Rahmen einer bevorzugten Ausführungsform einer solchen Bereitstellung ist das CO ein Verfahrensprodukt einer Umsetzung von zumindest Methan aus biologischer Quelle und Wasserdampf (besonders bevorzugt unter Zusatz von $CO_2$), unter Zuführung von Wärmeenergie bei einer Temperatur von mindestens 500°C zu Kohlenmonoxid. Ein darüber hinaus geeigneter, nachhaltiger Reformerprozess mit Zusatz von $CO_2$ wird in der PCT-Patentanmeldung mit der Anmeldenummer PCT/EP2022/052267 beschrieben, auf die ausdrücklich und vollinhaltlich Bezug genommen wird. Dieses Verfahren betrifft die Herstellung von Kohlenmonoxid aus Methan aus biologischer Quelle, Wasserdampf und $CO_2$, enthaltend zumindest die Schritte

Synthese von Kohlenmonoxid in einem Reformerprozess, worin Methan aus biologischer Quelle und Wasserdampf unter Zusatz von mindestens $CO_2$ unter Zuführung von Wärmeenergie bei einer Temperatur von mindestens 500°C zu Kohlenmonoxid-haltigem Produktgas umgesetzt wird,

Reinigung des aus vorgenannter Synthese erhaltenen Kohlenmonoxid-haltigen Produktgases, mindestens durch Abtrennung von $CO_2$ und gegebenenfalls zusätzlich durch mindestens eine Abtrennung ausgewählt aus der Abtrennung von Wasser, der Abtrennung von Wasserstoff oder einer Kombination davon, unter Erhalt von Kohlenmonoxid;

Bereitstellung von $CO_2$ für den besagten Zusatz zum vorgenannten Reformerprozess zumindest aus besagter Abtrennung von $CO_2$ des vorgenannten Reinigungsschritts.

Dabei ist es wiederum bevorzugt, wenn die zur Synthese von Kohlenmonoxid dem Reformerprozess zugeführte Wärmeenergie durch mindestens eine Methode bereitgestellt wird, ausgewählt aus (i) der Verbrennung von Brennstoff, der mittels regenerativer Energie erzeugten Wasserstoff enthält, (ii) der Verbrennung von Brennstoff, der Methan aus biologischer Quelle enthält, (iii) Umwandlung von aus regenerativer Energie erzeugter, elektrischer Energie in Wärme.

Unter "Methan aus biologischer Quelle" (auch als Bio-Methan bezeichnet), versteht der Fachmann in Abgrenzung zu fossilem Methan solches Methan, welches technisch durch Methan-Gärung aus Biomasse gewonnen wird. Die Methan-Gärung ist bekanntermaßen der anaerobe Abbau organischer Stoffe durch Mikroorganismen. Methan aus biologischer Quelle wird beispielsweise in Biogasanlagen hergestellt, worin sowohl organische Abfälle als auch nachwachsende Rohstoffe entsprechend vergoren werden.

Unter "regenerativer Energie" versteht der Fachmann Energie aus einer Energiequelle, die sich nicht erschöpft, wie z.B. Windenergie, Wasserenergie, Bioenergie (z.B. Verstromung von Biogas oder Biomasse) oder Sonnenenergie. Als regenerative Energie eignen sich daher ganz besonders bevorzugt wahlweise Windkraft, Sonnenenergie, Wasserkraft oder Mischungen daraus.

Erfindungsgemäß bevorzugt ist das gemäß i) des erfindungsgemäßen Verfahrensschritts a) bereitgestellte Methanol ein Verfahrensprodukt einer Umsetzung von CO, wobei dieses bei der Umsetzung eingesetzte CO ein Verfahrensprodukt mindestens einer partiellen Reduktion von $CO_2$ zu CO ist, insbesondere ausgewählt aus einer reverse water-gas-shift Reaktion unter Einsatz von mittels Elektrolyse (bevorzugt mittels Wasserelektrolyse, i.e. Elektrolyse von Wasser) bereitgestelltem $H_2$, aus einer elektrochemischen Reduktion von $CO_2$ zu CO oder aus Mischungen daraus.

Im Rahmen einer besonders bevorzugten Ausführungsform des erfindungsgemäßen Verfahrens wird das bereitgestellte Methanol als Verfahrensprodukt einer Umsetzung von CO bereitgestellt, wobei das bei der Umsetzung eingesetzte CO ein Verfahrensprodukt mindestens einer partiellen Reduktion eines Gasstroms hergestellt wurde, der mindestens $CO_x$ mit x = 1 oder 2 und gegebenenfalls Wasserstoffgas enthält.

Es hat sich weiter als ganz bevorzugt herausgestellt, wenn in einer Ausführungsform des Verfahrens Methanol durch die Umsetzung von $CO_2$ mit oder ohne Wasserstoff gewonnen und für die Bereitstellung von Methanol genutzt wird. Dabei ist es wiederum besonders bevorzugt, wenn die Umsetzung eine elektrochemische Reaktion, eine homogen katalysierte Reaktion oder eine heterogen katalysierte Reaktion ist. Das dafür eingesetzte $CO_2$ stammt in einer weiteren Ausführungsform zusätzlich aus einer weiteren $CO_2$-Quelle, beispielsweise das bei der Bereitstellung von Wärmeenergie emittierte $CO_2$ oder das $CO_2$ aus einer externen $CO_2$-Quelle, z.B. einem Industrieabgas.

Eine "elektrochemische Reaktion" findet erfindungsgemäß durch Anlegen von elektrischem Strom im Reaktionsmedium (z.B. über mindestens eine in das Reaktionsmedium eintauchende Elektrode) in Gegenwart mindestens eines Reaktanden (z.B. Kohlendioxid) statt.

Eine "katalysierte Reaktion" oder "katalytische Umsetzung" wird wie zuvor definiert (*vide supra*). Eine "homogen katalysierte Reaktion" findet erfindungsgemäß unter Einsatz eines homogenen Katalysators und eine "heterogen katalysierte Reaktion" unter Einsatz eines heterogenen Katalysators statt.

Als eine bevorzugte $CO_2$-Quelle dient mindestens eine "externe $CO_2$-Quelle", die $CO_2$ beiträgt, welches nicht durch das erfindungsgemäße Verfahren emittiert wird. Eine externe $CO_2$ Quelle wäre z.B. das $CO_2$, das bei einer Zementherstellung, bei einer $H_2$-Herstellung für die Ammoniaksynthese anfällt, bei einer Fermentation anfällt, bei Verbrennung von Brennstoffen (z.B. einer Abfallverbrennung) im Abgas entsteht, oder $CO_2$, das aus der Luft gewonnenen wird.

Im Rahmen einer besonders bevorzugten Ausführungsform des erfindungsgemäßen Verfahrens wird das Kohlenmonoxid für die Methanolsynthese aus $CO_2$ in einer reverse water-gas shift (RWGS) Reaktionszone hergestellt, durch mindestens eine Umsetzung von Wasserstoff und $CO_2$ zu Kohlenmonoxid. Ein solche Ausführungsform der Kohlenmonoxid-Herstellung wird beispielsweise in dem Dokument WO 2021/089737 A beschrieben, auf das hierin ausdrücklich und vollinhaltlich Bezug genommen wird.

Eine "Reaktionszone" ist der Teil eines Reaktionsraumes, in dem eine chemische Reaktion, z.B. die reverse water-gas shift Reaktion, abläuft. Ein "Reaktionsraum" ist ein Volumen, in dem die an einer chemischen Reaktion beteiligten Reaktionspartner zusammengebracht werden und in dem die chemische Reaktion stattfindet. Dies kann für eine chemische Reaktion beispielsweise das Volumen eines Gefäßes sein, in dem sich ein Edukt, z.B. im Falle einer RWGS-Reaktion Kohlendioxid, und dessen Reaktionspartner, im Falle einer RWGS-Reaktion Wasserstoff, gemeinsam befinden und in der Reaktionszone zur Reaktion gebracht werden. Dieses Volumen kann sich beispielsweise in einem Reaktor befinden.

Das für die RWGS-Reaktion genutzte Wasserstoffgas wird bevorzugt mittels Elektrolyse, insbesondere mittels Chloralkalielektrolyse oder Wasserselektrolyse, besonders bevorzugt mittels Wasserelektrolyse, bereitgestellt. Dabei wird wiederum jeweils bevorzugt aus regenerativer Energie (insbesondere aus Wasserkraft, Sonnenenergie oder Windkraft) hergestellte elektrische Energie verwendet.

In dem besonders bevorzugten Verfahren ist das für die Bereitstellung des Methanols bereitgestellte Kohlenmonoxid ein Produkt eines Verfahrens mit mindestens folgenden Schritten:

Bereitstellung eines $CO_2$-Gasstroms,

Reinigung des $CO_2$ Gasstroms von Nebenbestandteilen, insbesondere von Stickoxiden, Schwefelverbindungen, Staub, Wasser, Sauerstoff und HCl wahlweise mittels Adsorption, Gaswäsche oder katalytischer Behandlung unter Erhalt eines gereinigten Kohlendioxids,

Einspeisung von Wasserstoffgas zusammen mit dem gereinigten $CO_2$ Gasstrom in eine RWGS Reaktionszone und Umsetzung der Edukte nach dem Prinzip der RWGS Reaktion zu einem Produktgasgemisch aus Wasserdampf, CO und gegebenenfalls Nebenprodukten, insbesondere niedere Kohlenwasserstoffe, insbesondere bevorzugt Methan,

Abtrennung von nicht umgesetztem Kohlendioxid aus dem aus der Abtrennung erhaltenen Gasgemischs der RWGS Reaktion, insbesondere mittels Aminwäsche, und Rückführung des nicht umgesetzten Kohlendioxids in die RWGS Reaktion,

Abtrennung des in der RWGS Reaktion nicht umgesetzten Wasserstoffs aus dem nach der Abtrennung erhaltenen Gasgemisch von Kohlenmonoxid und Wasserstoff, insbesondere unter Verwendung einer Coldbox, und wahlweise Rückführung des Wasserstoffs in die RWGS Reaktion,

Ausbringung des verbliebenen Kohlenmonoxids aus der Abtrennung.

Als $CO_2$-Quelle zur Bereitstellung des $CO_2$-Gasstroms dient beispielsweise das bei der Bereitstellung von Wärmeenergie für das erfindungsgemäße Verfahren (z.B. für die RWGS-Reaktion) emittierte $CO_2$, das bei einer Luftzerlegung erhaltene $CO_2$ oder $CO_2$ aus einer weiteren externen $CO_2$-Quelle.

Eine "externe $CO_2$-Quelle" trägt $CO_2$ bei, welches nicht durch das erfindungsgemäße Verfahren oder dessen Ausführungsformen emittiert wird. Eine externe $CO_2$ Quelle wäre z.B. das $CO_2$, das bei einer Zementherstellung, bei Verbrennung von Brennstoffen (z.B. einer Abfallverbrennung) im Abgas entsteht, oder $CO_2$, das aus der Luft gewonnenen wird. Dieses $CO_2$ aus einer externen $CO_2$-Quelle wird im Rahmen einer bevorzugten Ausführungsform des erfindungsgemäßen Verfahrens durch Absorption eines $CO_2$ -Anteils aus (i) Prozessgasen oder Abgasen, die aus mindestens einem Prozess ausgewählt aus Zementherstellung, $H_2$-Herstellung, Verbrennung ausgewählt wird und/oder (ii) aus Luft durch einleiten in Alkalilauge, zum Beispiel Kalilauge, erfolgen. Hierbei bildet sich Kaliumhydrogencarbonat, welches anschließend wieder zu $CO_2$ und Kalilauge thermisch zersetzt werden kann. Das dabei freigesetzte $CO_2$ wird dann dem erfindungsgemäßen Prozess zur Synthese von Kohlenmonoxid zugeführt.

Die RWGS Reaktion wird in der Reaktionszone bevorzugt bei einer Temperatur $\geq$ 650°C, besonders bevorzugt $\geq$ 700°C, ganz besonders bevorzugt $\geq$ 750°C durchgeführt.

Die RWGS Reaktion wird bevorzugt in Gegenwart mindestens eines Katalysators durchgeführt. Dieser ist besonders bevorzugt ausgewählt aus mindestens einer Verbindung aus der Gruppe:

(I) Mischmetalloxide der Formel $A_{(1-w-x)}A'_wA''_xB_{(1-y-z)}B'_yB''_zO_{3-delta}$ wobei hier gilt:

A, A' und A" sind unabhängig voneinander ausgewählt aus der Gruppe: Mg, Ca, Sr, Ba, Li, Na, K, Rb, Cs, Sn, Sc, Y, La, Ce, Pr, Nd, Pm, Sm, Eu, Gd, Tb, Dy, Ho, Er, Tm. Yb, Tl , Lu, Ni, Co, Pb, Bi und/oder Cd; und

B, B' und B" sind unabhängig voneinander ausgewählt aus der Gruppe: Cr, Mn, Fe, Bi, Cd, Co, Cu, Ni, Sn. Al, Ga, Sc, Ti, V, Nb, Ta, Mo, Pb. Hf, Zr, Tb. W, Gd. Yb, Mg, Li, Na, K, Ce und/oder Zn; und

$0 \leq w \leq 0.5$; $0 \leq x < 0.5$; $0 \leq y \leq 0.5$; $0 \leq z \leq 0.5$ und $-1 \leq delta \leq I$;

(II) Mischmetalloxide der Formel $A_{(1-w-x)}A'_wA''_xB_{(1-y-z)}B'_yB''_zO_{3-delta}$ wobei hier gilt:

A, A' und A" sind unabhängig voneinander ausgewählt aus der Gruppe: Mg, Ca, Sr, Ba. Li, Na, K, Rb, Cs, Sn, Sc, Y, La, Ce, Pr, Nd, Sm, Eu, Gd, Tb, Dy, Ho, Er, Tm, Yb, Tl, Lu, Ni, Co, Pb und/oder Cd; und

B ist ausgewählt aus der Gruppe: Cr, Mn, Fe, Bi, Cd, Co, Cu, Ni, Sn, Al, Ga, Sc, Ti, V, Nb, Ta, Mo, Pb, Hf, Zr, Tb, W, Gd, Yb, Mg, Cd, Zn, Re, Ru, Rh, Pd, Os, Ir und/oder Pt; und

B' ist ausgewählt aus der Gruppe: Re, Ru, Rh, Pd, Os, Ir und/oder Pt; und B" ist ausgewählt aus der Gruppe: Cr, Mn, Fe, Bi, Cd, Co, Cu, Ni, Sn, Al, Ga, Sc, Ti, V, Nb, Ta, Mo, Pb, Hf, Zr, Tb. W. Gd, Yb. Mg, Cd und/oder Zn; und

$0 \leq w \leq 0{,}5$; $0 \leq x \leq 0{,}5$; $0 \leq y \leq 0{,}5$; $0 \leq z \leq 0.5$ und $-I \leq delta \leq 1$ ;

(III) Mischungen von wenigstens zwei verschiedenen Metallen M1 und M2 auf einem Träger, welcher ein mit einem Metall M3 dotiertes Oxid von Al, Ce und/oder Zr umfasst;

wobei hier gilt: M1 und M2 sind unabhängig voneinander ausgewählt aus der Gruppe: Re, Ru, Rh, Ir, Os, Pd und/oder Pt; und

M3 ist ausgewählt aus der Gruppe: Sc, Y, La, Ce, Pr, Nd, Sm, Eu, Gd, Tb, Dy, Ho, Er, Tm, Yb und/oder Lu;

(IV) Mischmetalloxide der Formel $LO_x(M_{(y/z)}Al_{(2-y/z)}O_3)_z$; wobei hier gilt:

L ist ausgewählt aus der Gruppe: Na, K, Rb, Cs, Mg, Ca, Sr, Ba, Sc, Y, Sn, Pb, Pd, Mn, In, Tl, La, Ce, Pr, Nd, Sm, Eu, Gd, Tb, Dy, Ho, Er, Tm. Yb und/oder Lu; und

M ist ausgewählt aus der Gruppe: Ti, Zr, Hf, V, Nb, Ta, Cr, Mo, W, Mn, Re, Fe, Ru, Os, Co, Rh. Ir, Ni, Pd. Pt. Zn, Cu, Ag und/oder Au; und

$1 < x \leq 2$; $0 < y \leq 12$; und $4 \leq z \leq 9$;

(V) Mischmetalloxide der Formel $LO(Al_2O_3)_z$; wobei hier gilt:

L ist ausgewählt aus der Gruppe: Na, K, Rb. Cs, Mg, Ca, Sr, Ba, Sc, Y, Sn, Pb, Mn, In, Tl, La, Ce, Pr, Nd, Sm, Eu, Gd, Tb, Dy, Ho, Er, Tm, Yb und/oder Lu; und

$4 \leq z \leq 9$;

(VI) oxidischer Katalysator, der Ni und Ru umfasst.

(VII) Metall M1 und/oder wenigstens zwei verschiedene Metalle M1 und M2 auf und/oder in einem Träger, wobei der Träger
ein Carbid, Oxycarbid, Carbonitrid, Nitrid, Borid, Silicid, Germanid und/oder Selenid der Metalle A und/oder B ist; wobei hier gilt:

M1 und M2 sind unabhängig voneinander ausgewählt aus der Gruppe: Cr, Mn, Fe, Co, Ni, Re, Ru, Rh, Ir, Os, Pd, Pt, Zn, Cu, La, Ce, Pr, Nd, Sm, Eu, Gd, Tb, Dy, Ho, Er, Tm, Yb, und/oder Lu; und

A und B sind unabhängig voneinander ausgewählt aus der Gruppe: Be, Mg, Ca, Sc, I i, V, Cr, Mn, Fe, Co, Ni, Y, Zr, Nb, Mo, Hf, Ta, W, La, Ce, Pr, Nd, Sm, Eu, Gd, Tb, Dy, Ho, Er, Tm, Yb, und/oder Lu;

und/oder
Reaktionsprodukte von (I), (II), (III), (IV), (V), (VI) und/oder (VII) in Gegenwart von Kohlendioxid, Wasserstoff, Kohlenmonoxid und/oder Wasser bei einer Temperatur von ≥ 700 °C.

Das für die Bereitstellung des Hexamethylendiamins in dem erfindungsgemäßen Verfahren bereitgestellte Methanol wird in einer ebenso bevorzugten Ausführungsform des Verfahrens aus Kohlenmonoxid bereitgestellt, wobei das Kohlenmonoxid ein Produkt einer elektrochemischen partiellen Reduktion von Kohlendioxid zu Kohlenmonoxid ist. Im Rahmen dieser Ausführungsform wird zur CO Bereitstellung ein $CO_2$ Gasstrom in eine Elektrolysevorrichtung eingebracht und an einer Elektrode, bevorzugt an einer Gasdiffusionselektrode, (besonders bevorzugt unter Einsatz von aus regenerativer Energie hergestellter elektrischer Energie) zu Kohlenstoffmonoxid reduziert. Diese elektrochemische partielle Reduktion wird nachfolgend auch mit $CO_2$-Elektrolyse bezeichnet.

Die $CO_2$-Elektrolyse kann beispielsweise eine Hochtemperaturelektrolyse sein, die bei einer Temperatur von mehr als 600°C ggf. auch unter Zusatz von Wasser zur Herstellung von Synthesegas, betrieben wird. Hochtemperaturelektrolysen sind grundsätzlich bekannt und im Markt verfügbar z.B. von Haldor Topsoe, eCOs®. Bei der Hochtemperaturelektrolyse entsteht an der Anode Sauerstoff.

Wird die $CO_2$-Elektrolyse als Niedertemperaturelektrolyse betrieben, so erfolgt die Elektrolyse bei einer Temperatur unter 150°C.

Bei allen $CO_2$-Elektrolysen wird das $CO_2$ Gas dem Kathodenraum zugeführt.

Im Falle der Niedertemperaturelektrolyse wird $CO_2$ insbesondere an einer Gasdiffusionselektrode zu Kohlenmonoxid und ggf. Wasserstoff umgesetzt. Der Fachmann kennt beispielsweise aus der Druckschrift WO 2021/069470 A Elektroden und eine Methode zur Ausführung einer elektrochemischen Reduktion von $CO_2$. Bevorzugterweise wird die elektrochemische Reduktion von $CO_2$ nach einem Verfahren der WO 2021/069470 A durchgeführt. Auf diese Druckschrift wird hierin ausdrücklich und vollinhaltlich Bezug genommen. In diesem bevorzugten elektrolytischen Verfahren zu Herstellung von Kohlenmonoxid werden Kohlenmonoxid, ggf. Wasserstoff und Chlor durch elektrochemische Umsetzung von Kohlendioxid und Alkalichloridlösung erhalten. Dieses bevorzugte Elektrolyseverfahren ist dadurch gekennzeichnet, dass das Kohlendioxid an einer Gasdiffusionselektrode als Kathode in einer wässrigen Alkalichlorid-haltigen Lösung als Katholyt elektrochemisch reduziert und gleichzeitig Chlor aus einer wässrigen Alkalichlorid-haltigen Lösung als Anolyt anodisch erzeugt wird, wobei das im Katholyt gebildete Alkalisalz der Kohlensäure, ausgewählt aus Alkalicarbonat, Alkalihydrogencarbonat oder Mischungen daraus anschließend mit Chlorwasserstoff zu Kohlendioxid und Alkalichlorid umgesetzt wird und das hierbei freigesetzte Kohlendioxid in den Kathodenraum zur Gasdiffusionselektrode und das erzeugte Alkalichlorid wahlweise in den Anodenraum und/oder in den Kathodenraum zurückgeführt werden.

Nach den bekannten Prinzipien kann bei der Niedertemperatur-Elektrolyse auch ein MEA (Membran-Electrode-Assembly) Konzept zum Einsatz kommen. Hierbei wird auf die Membran ein Katalysator aufgebracht. Eine davor gelagerte Gasdiffusionsschicht reguliert den Gas- und Flüssigkeitstransport. Dies kann sowohl auf der Anoden- als auch auf der Kathodenseite erfolgen. Weiterhin besteht die Möglichkeit eine Gasdiffusionselektrode in direktem Kontakt mit der Membran zu bringen.

Die eingesetzte Gasdiffusionselektrode kann in einer zero-gap als auch in einer finite-gap Anordnung in der Elektrolysezelle verbaut sein. Eine bevorzugte Anordnung für eine Niedertemperaturelektrolyse von $CO_2$ wird in der Druckschrift WO 2020/057998 A1 beschrieben, auf die ausdrücklich und vollinhaltlich Bezug genommen wird.

Dem Kathodenraum, bzw. der darin installierten Gasdiffusionselektrode, kann ein Überschuss an $CO_2$ zugeführt werden. Überschuss bedeutet, mehr $CO_2$ einzuleiten als für die stöchiometrische Umsetzung aufgrund des fließenden elektrischen Stromes notwendig wäre. Somit gelangt aus dem Kathodenraum eine Gasmischung bestehend aus nicht umgesetzten $CO_2$, CO und $H_2$.

Im Rahmen einer weiteren Ausführungsform der $CO_2$-Elektrolyse ist es bevorzugt, wenn die dafür verwendete elektrische Energie aus regenerativer Energie hergestellte elektrische Energie, insbesondere aus Windkraft, Sonnenenergie oder Wasserkraft hergestellte elektrische Energie, ist.

Sofern das für die Bereitstellung des Methanols genutzte Kohlenmonoxid ein Verfahrensprodukt mindestens eines der vorgenannten Verfahren ist, kann für die Umsetzung des CO zu Methanol mindestens eines der dem Fachmann dafür bekannten Verfahren ausgehend von CO-haltigem Synthesegas genutzt werden.

Sollte aus Schritt ii-1) nicht ohnehin direkt CO im Gemisch mit ausreichend Wasserstoff als Synthesegas entstehen, so wird bei Mangel an einer ausreichenden Menge Wasserstoffgas dem CO vor der Umsetzung zu Methanol Wasserstoffgas unter Erhalt von Synthesegas beigemischt. Dieses im Rahmen einer Bereitstellung von Methanol dem CO zusätzlich beigefügte Wasserstoffgas wird bevorzugt durch Elektrolyse, insbesondere durch Chloralkalielektrolyse oder Wasserelektrolyse, bereitgestellt. Die Wasserelektrolyse kann mit Anlagen nach dem Stand der Technik durchgeführt werden. Bekannt und kommerziell erhältlich sind technische Anlagen zur alkalischen Wasserelektrolyse als auch zur Polymerelektrolyt-basierten Elektrolyse, der so genannten PEM-Elektrolyse. Die Prinzipien der Wasserelektrolyse sind beispielhaft in Kapitel 6.3.4 in Volkmar M. Schmidt in "Elektrochemische Verfahrenstechnik" (2003 Wiley-VCH-Verlag; ISBN 3-527-29958-0) beschrieben. Im Rahmen einer weiteren Ausführungsform ist es bevorzugt, wenn die für die Elektrolyse zur Wasserstoffherstellung verwendete elektrische Energie aus regenerativer Energie hergestellte elektrische Energie, insbesondere aus Windkraft, Sonnenenergie oder Wasserkraft hergestellte elektrische Energie, ist.

Besonders bevorzugt ist eine Ausführungsform, worin das für das erfindungsgemäße Verfahren bereitzustellende Methanol entsprechend als Verfahrensprodukt unter Verwendung regenerativer Energie hergestellt wurde, vorzugsweise an einem geografischen Ort mit guter Verfügbarkeit von regenerativer Energie, um anschließend entweder dieses Methanol durch Lieferung per Transport in Behältern oder per Methanolstrom im Rahmen eines kontinuierlichen Prozesses für das erfindungsgemäße Verfahren bereitzustellen. Für die Bereitstellung im Rahmen eines kontinuierlichen Prozesses steht die Methanolherstellung bevorzugt in Fluidverbindung mit der Herstellung des Hexamethylendiamins, z.B. über eine Rohrleitung.

Anlagen für die Methanolproduktion können am Markt lizenziert werden, beispielsweise bei der Firma Air Liquide, Johnson Matthey und bei weiteren Firmen. Eine Übersicht zur klassischen Methanolproduktion aus Synthesegas ist beispielsweise in Ott et al., Methanol in: Ullmann's Encyclopedia of industrial chemistry, 2012, Wiley-VCH Verlag, Weinheim (doi 10.1002/14356007.a16_465.pub3) publiziert, auf die ausdrücklich und vollinhaltlich Bezug genommen wird.

Das wie zuvor beschrieben gemäß Schritt a) i) bereitgestellte Methanol wird in Schritt a) ii) des erfindungsgemäßen Verfahrens zur Bereitstellung des Hexamethylendiamins durch ein Verfahren, enthaltend zumindest folgende Schritte, zu Propen umgesetzt:
Bereitstellung von Propen als Produkt eines Verfahrens mit zumindest folgenden Schritten:

ii-1) Umsetzung des zuvor bereitgestellten Methanols zu einer Produktmischung, enthaltend Dimethylether, Wasser und Methanol;

ii-2) Umwandlung von Ausgangsmaterial, enthaltend Dimethylether, Wasser und Methanol jeweils aus der vorgenannten Produktmischung, bei einer Temperatur von mehr als 200°C durch Kontakt mit einem Katalysator, vorzugsweise mit mindestens einer Zeolith-Verbindung, zu Propen.

Die Schritte ii-1) und ii-2) sind Teil des Methanol-to-Propylene-Prozesses (auch MTP-Prozess genannt). Der MTP-Prozess wurde zuerst von der Firma Lurgi entwickelt.

In einem MTP-Prozess wird beispielsweise gasförmiges Methanol in einem Reaktor an einem Katalysator zu einer Produktmischung enthaltend Dimethylether, Wasser und Methanol im Sinne des Schritts ii-1) des erfindungsgemäßen Verfahrens umgesetzt. Als Reaktor eignen sich dafür beispielsweise ein Festbett-Reaktor oder ein Wirbelschichtreaktor.

Im Rahmen einer bevorzugten Ausführungsform des erfindungsgemäßen Verfahrens, erfolgt die Umsetzung des bereitgestellten Methanols gemäß Schritt a) ii-1) durch Kontaktieren des Methanols, bevorzugt des Methanols in der Gasphase, mit einem Katalysator.

Als besonders bevorzugter Katalysator zur Umwandlung des Methanols in Schritt ii-1) eignet sich beispielsweise $Al_2O_3$-Granulat, wie beschrieben in den Druckschriften EP 0 448 000 B1 oder DE 197 23 363 A1.

Vor der Umwandlung des Methanols in Schritt a) ii-1) wird das bereitgestellte Methanol bevorzugt auf eine Temperatur in einem Bereich von 200 °C bis 350°C gebracht und die Umwandlung mit diesem entsprechend temperierten Methanol durchgeführt. Die Produktmischung enthaltend Dimethylether, Methanol und Wasser weist nach der Umwandlung wiederum bevorzugt eine Temperatur von 350°C bis 450°C auf.

Die Produktmischung aus Schritt ii-1), insbesondere in Form eines Gasstroms, wird, gegebenenfalls nach der Durchführung von optionalen Reinigungsschritten, als Ausgangsmaterial für Schritt ii-2) beispielsweise in einen weiteren Reaktor eingebracht und dort an einem Katalysator zu Propen umgesetzt. Als Katalysator eignet sich beispielsweise der von der Firma Clariant unter dem Handelsnamen MTPROP® vertriebenen Katalysator, oder eine Zeolith-Verbindung als Katalysator, wie er beispielsweise in der Druckschrift US 7,015,369 B2 in Spalte 1, Zeilen 43-52, sowie in deren Beispielen 1 und 2 beschrieben ist. Vorzugsweise wird im Rahmen einer weiteren Ausführungsform des erfindungsgemäßen Verfahrens das Ausgangsmaterial durch Kontakt an einer Zeolith-Verbindung als Katalysator zu Propen umgewandelt.

Es ist bevorzugt, wenn in Schritt ii-2) das Ausgangsmaterial bei einer Temperatur von 350°C bis 600°C, insbesondere bei einer Temperatur von 380°C bis 550°C, umgesetzt wird.

Das durch Schritt ii-2) des erfindungsgemäßen Verfahrens bereitgestellte Propen wird, gegebenenfalls nach der Abtrennung von Nebenbestandteilen, gemäß Schritt iii) mit Ammoniak zu Acrylnitril umgesetzt. Für die Bereitstellung des Hexamethylendiamins ist es erfindungsgemäß ausreichend, wenn das dafür eingesetzte Acrylnitril ein tatsächliches Verfahrensprodukt mindestens der unter a) genannten Schritte i), ii) und iii) ist. Dies bedeutet, dass es für die Ausführung des Schrittes der Bereitstellung des Hexamethylendiamins bereits ausreicht, das auf besagte Weise hergestellte Acrylnitril lediglich im Rahmen einer Belieferung als Rohstoff aus einem Lagerbehälter oder aus einer Zuleitung zu entnehmen, um dieses zumindest dem Schritt a) iv) des erfindungsgemäßen Verfahrens zuzuführen. In diesem Falle führt der Hexamethylendiisocyanat-Produzent als Ausführender des erfindungsgemäßen Verfahrens die unter a) des erfindungsgemäßen Verfahrens genannten Schritte i), ii) und iii) zur Herstellung des Acrylnitrils nicht selbst aus, sondern er sorgt nur dafür, dass das bereitgestellte Hexamethylendiamin aus Acrylnitril hergestellt wurde, das durch Anwendung mindestens der unter a) genannten Schritte i), ii) und iii) synthetisiert wurde und somit deren tatsächliches Verfahrensprodukt ist.

Für die Synthese von Acrylnitril aus dem Propen wird zunächst Ammoniak als tatsächliches Verfahrensprodukt eines Verfahrens mit mindestens folgenden Schritten bereitgestellt:

iii-1-1) Bereitstellung von Wasserstoffgas durch Elektrolyse von Wasser, bevorzugt unter Einsatz von aus regenerativer Energie erzeugter elektrischer Energie;

iii-1-2) Umsetzung des bereitgestellten Wasserstoffgases mit gasförmigem Stickstoff zu Ammoniak.

Die in Schritt iii-1-1) zur Synthese des bereitzustellenden Wasserstoffgases auszuführende Elektrolyse von Wasser lässt sich mit Anlagen nach dem Stand der Technik durchführen. Bekannt und kommerziell erhältlich sind technische Anlagen zur alkalischen Wasserelektrolyse als auch zur Polymerelektrolyt-basierten Elektrolyse, der so genannten PEM-Elektrolyse. Die Prinzipien der Wasserelektrolyse sind beispielhaft in Kapitel 6.3.4 in Volkmar M. Schmidt in "Elektrochemische Verfahrenstechnik" (2003 Wiley-VCH-Verlag; ISBN 3-527-29958-0) beschrieben.

Die Herstellung des Ammoniaks erfolgt gemäß Schritt a) iii-1-2) ausgehend von Stickstoff und dem im vorherigen Schritt a) iii-1-1) bereitgestelltem Wasserstoff in einer dem Fachmann notorisch bekannten Weise, wie beispielsweise nach dem Haber-Bosch-Verfahren unter Anwendung bekannter Reaktortechnologien und -designs für dieses Verfahren beschrieben, auf die hier ausdrücklich und vollinhaltlich Bezug genommen wird.

Für die Bereitstellung bzw. die Herstellung des Ammoniaks hat es sich als bevorzugt erwiesen, wenn der dafür genutzte Stickstoff durch eine Luftzerlegung bereitgestellt wird. Prozesse zur Luftzerlegung und entsprechend geeignete Anlagen zur Stickstoffherstellung gehören zum Stand der Technik und sind auf dem Markt erhältlich.

Das durch zumindest vorgenannte Verfahrensschritte bereitgestellte Propen wird sodann mit dem bereitgestellten Ammoniak oxidativ zu Acrylnitril, bevorzugt nach dem sogenannten SOHIO-Verfahren (beispielsweise gemäß US 2904580 A), umgesetzt. Dabei ist es erfindungsgemäß bevorzugt, wenn die Umsetzung des bereitgestellten Ammoniaks mit dem Propen gemäß Schritt a) iii-2) unter Einsatz von Luftsauerstoff bei einer Temperatur von 350°C bis 550°C erfolgt.

Im Rahmen einer weiteren Ausführungsform des Verfahrens zu Herstellung des bereitgestellten Acrylnitrils wird die Umsetzung besonders bevorzugt bei einem relativen Druck von 40 bis 220 kPa.

Es hat sich als vorteilhaft erwiesen, die Umsetzung des bereitgestellten Ammoniaks mit dem Propen gemäß Schritt a) iii-2) an einem Molybdat- und/oder Antimonat-basierten Katalysator erfolgen zu lassen. Im Rahmen dieser Ausführungsform ist es wiederum bevorzugt, wenn die vorgenannte Temperatur eingestellt wird und die Umsetzung unter Einsatz von Sauerstoff, bevorzugt Luftsauerstoff, erfolgt.

Das gemäß Schritt iii-2) des erfindungsgemäßen Verfahrens hergestellte Acrylnitril wird gemäß Schritt iv) elektrochemisch zu Adiponitril (CAS Nr. 111-69-3, auch als Adipinsäuredinitril bezeichnet) umgesetzt. Für die Bereitstellung des Hexamethylendiamins ist es erfindungsgemäß ausreichend, wenn das dafür eingesetzte Adiponitril ein tatsächliches Verfahrensprodukt mindestens der unter a) genannten Schritte i), ii), iii) und iv) ist. Dies bedeutet, dass es für die Ausführung des Schrittes der Bereitstellung des Hexamethylendiamins bereits ausreicht, das auf besagte Weise hergestellte Adiponitril lediglich im Rahmen einer Belieferung als Rohstoff aus einem Lagerbehälter oder aus einer Zuleitung zu entnehmen, um dieses zumindest dem Schritt a) v) des erfindungsgemäßen Verfahrens zuzuführen. In diesem Falle führt der Hexamethylendiisocyanat-Produzent als Ausführender des erfindungsgemäßen Verfahrens die unter a) des erfindungsgemäßen Verfahrens genannten Schritte i), ii) und iii) und iv) zur Herstellung des Adiponitrils nicht selbst aus, sondern er sorgt nur dafür, dass das bereitgestellte Hexamethylendiamin aus Adiponitril hergestellt wurde, das durch Anwendung mindestens der unter a) genannten Schritte i), ii), iii) und iv) synthetisiert wurde und somit deren tatsächliches Verfahrensprodukt ist.

Das besagte Adiponitril muss ein tatsächliches Verfahrensprodukt zumindest der Schritte

Einbringung einer Mischung, enthaltend Wasser, besagtes Acrylnitril aus Schritt a) iii-2) und mindestens ein Elektrolytsalz in den Kathodenraum einer Elektrolysezelle und Inkontaktbringen der Mischung mit einer Kathode, an der elektrischer Strom anliegt;

kathodische Hydrodimerisierung des Acrylnitrils unter Bildung einer Adiponitril-haltigen Produktmischung;

Ausbringung der Adiponitril-haltigen Produktmischung aus dem Kathodenraum der Elektrolysezelle und optional Aufreinigung des Adiponitrils;

sein. Diese Schritte werden beispielsweise wie in dem Schutzrecht US 3,193,480 A beschrieben ausgeführt, auf welches hier ausdrücklich und vollinhaltlich Bezug genommen wird.

Zur Bereitstellung der Mischung werden mindestens Wasser, besagtes Acrylnitril und mindestens ein Elektrolytsalz vermengt. Dabei bildet sich bevorzugt eine Emulsion von Acrylnitril-haltigen Tröpfchen in Wasser.

Als Elektrolytsalz im Kathodenraum der Elektrolysezelle eignet sich hierfür im Rahmen einer bevorzugten Ausführungsform mindestens ein quartäres Ammoniumsalz, insbesondere mindestens ein quartäres Ammoniumsulfonat. Besonders bevorzugtes Elektrolytsalz wird ausgewählt aus mindestens einem Elektrolytsalz aus der Gruppe, die gebildet wird aus Tetraalkylammoniumsalzen, Tetraalkanolammoniumsalzen, Dialkyldialkanolammoniumsalzen, Alkyltrialkanolammoniumsalzen, Trialkylalkanolammonium-salzen, Trialkylbenzylammoniumsalzen, quartären N-Heterocyclo-N-alkylammoniumsalzen, wobei die Sulfonsäure Salze hiervon wiederum weiter bevorzugt geeignet sind. Als ganz besonders bevorzugte Alkylgruppen der Ammoniumkationen vorgenannter Salze gelten ($C_1$-$C_6$)-Alkylgruppen wie Methyl, Ethyl, n-Propyl, Isopropyl, n-Butyl, sec-Butyl, iso-Butyl, tert-butyl. Als ganz besonders bevorzugte Alkanolgruppen gelten ($C_1$-$C_6$)-Hydroxyalkylgruppen wie 2-Hydroxyethyl, 2-Hydroxypropyl, 3-Hydroxypropyl, 4-Hydroxypropyl. Als besonders bevorzugte N-Heterocyclo-Gruppe eignen sich beispielsweise Piperidinium, Morpholinium, Pyrrolidinium.

Als bevorzugt geeignete Anionen der quartären Ammoniumsalze gelten Chlorid, Fluorid, Sulfate, Phosphate und Sulfonate.

Die Tetraalkylammoniumsalze von Aryl- oder Alkylarylsulfonsäuren sind besonders bevorzugt geeignete Elektrolytsalze. Besonders bevorzugte Sulfonat-Anionen der als Elektrolytsalz bevorzugt nutzbaren quartären Ammoniumsalze sind Benzolsulfonat, o-Toluylsulfonat, m-Toluylsulfonat, o-Toluylsulfonat, m-Toluylsulfonat, p-Toluylsulfonat, o-Cumylsulfonat, m-Cumylsulfonat, p-Cumylsulfonat, alpha-Naphthylsulfonat, beta-Naphthylsulfonat, p-Xylylsulfonat oder Mischungen daraus. Besonders bevorzugte Sulfat-Anionen sind ausgewählt aus mindestens einem Anion mit organischem Rest, der analog zu den organischen Resten der vorgenannten Sulfonat-Anionen ist. Im Rahmen einer äußerst bevorzugten Ausführungsform wird mindestens ein Elektrolytsalz ausgewählt aus Tetramethylammoniumchlorid, Tetraethylammoniumchlorid, Tetra(n-butyl)ammoniumchlorid, Tetraethylammonium-p-toluylsulfonat, Tetraethylammonium-o-toluylsulfonat, Tetraethylammonium-m-toluylsulfonat, Tetraethylammoniumbenzolsulfonat; Tetraethylammonium o-, m- oder p-Cumylsulfonat oder o-, m-, oder p-ethylbenzolsulfonat; N, N-Dimethylpiperidinium, o-, m- oder p-toluylsulfonat oder o-, m- oder p-bipyhenylsulfonat; Tetrabutylammoniun alpha- oder beta-naphthylsulfonat oder o-, m- or p-toluylsulfonat; Tetrapropylammonium o-, m- oder p-amylbenzolsulfonat oder alpha-ethyl-beta-naphthylsulfonate; Tetra(2-hydroxylethyl)ammonium o-, m- oder p-cumylsulfonat oder o-, m- oder p-toluylsulfonat; Tetra(4-Hydroxybutyl)ammonium benzolsulfonat oder p-xylyl-3-sulfonat; Tetrapentylammonium o-, m- oder p-toluylsulfonat oder o-, m- oder p-hexylbenzolsulfonate, Tetrapentanolammonium p-cumyl-3-sulfonat oder benzolsulfonat; Methyltriethylammonium o-, m- oder p-toluylsulfonat oder mesityl-2-sulfonat; Trimethylethylammonium o-xylyl-4-sulfonat oder o-, m- oder p-toluylsulfonat; Triethylpentylammonium alpha- oder beta-naphthylsulfonat oder o-, m- oder p-butylbenzolsulfonat, Trimethyl-Hydroxyethylammonium benzolsulfonat oder o-, m- oder p-toluylsulfonat; N, N-Diethylpiperidinium oder N-Methylpyrrolidinium o-, m- oder p-hexylbenzolsulfonate oder o-, m- oder p-toluylsulfonat, N, N-Di-isopropyl oder N, N-Dibutylmorpholinium, o-, m- oder p-toluylsulfonat oder o-, m- oder p-biphenylsulfonat, oder Mischungen aus den vorgenannten Salzen.

Im Rahmen einer weiteren bevorzugten Ausführungsform hat es sich als vorteilhaft erwiesen, wenn die in Schritt a) iv) die eingebrachte Mischung einen pH-Wert im Bereich von pH 7 bis pH 10, insbesondere von pH 7 bis pH 9,5, aufweist. Es ist weiter besonders bevorzugt, wenn der pH-Wert der Mischung im Kathodenraum über die Normalität des Anolyten im Anodenraum der Elektrolysezelle und der formalen Migration von $H^+$-Ionen von dem Anodenraum in den Kathodenraum gesteuert wird. Zu diesem Zweck weist der Anolyt im Anodenraum der Elektrolysezelle bevorzugt eine Normalität im Bereich von 0.4 bis 1.2 auf.

Die Elektrolysezelle, in der die elektrochemische Hydrodimerisierung stattfindet, weist in einer bevorzugten Ausführungsform einen Kathodenraum und einen Anodenraum auf, die durch eine Membran voneinander getrennt sind. Als Membran eignen sich hierfür beispielsweise die in der Druckschrift US 3,193,480 A genannten Membranen, bzw. sind kommerziell erhältlich, z.B. unter dem Handelsnamen Nafion® 115.

Es ist im Rahmen einer weiteren Ausführungsform des Verfahrens bevorzugt, wenn an der Elektrolysezelle ein elektrischer Strom mit einer Stromdichte von 15 bis 40 A/$dm^2$, besonders bevorzugt bei einer Spannung von 5 bis 20 Volt, anliegt.

Es ist erfindungsgemäß weiter bevorzugt, wenn die kathodische Hydrodimerisierung des Acrylnitrils zu Adiponitril durch einen anliegenden elektrischem Strom durchgeführt wird, der tatsächlich unter Einsatz von regenerativer Energie, insbesondere ausgewählt aus Windkraft, Wasserkraft, Sonnenenergie oder Mischungen daraus, erzeugt wurde.

Das gemäß Schritt a) iv) bereitgestellte Adiponitril wird durch eine katalytische Hydrierung mit Wasserstoff zu Hexamethylendiamin nach dem Fachmann bekannten Verfahren (bevorzugt bei einer Temperatur im Bereich von 100°C bis 200°C und einem operativen Druck im Bereich von 28 bis 41 MPa), umgesetzt. Die Hydrierung wird weiter bevorzugt in flüssiger Phase durchgeführt, wobei optional Ammoniak als Medium zum Wärmetransfer zugesetzt wird. Als Katalysatoren eignen sich für die katalytische Hydrierung von Adiponitril insbesondere Cobalt-haltige Katalysatoren, wie sie beispielsweise in US 3,232,888 A, US 3,821,305 A, US 3,773,832 oder US 4,598,058 A beschrieben werden. Ebenso nutzbar sind Eisen-haltige Katalysatoren, wie sie beispielsweise in US 3,696,153 A oder US 4,587,228 A beschrieben werden.

In Schritt b) des erfindungsgemäßen Verfahrens wird Phosgen hergestellt. Hierbei wird in Schritt b) i) des erfindungsgemäßen Verfahrens zunächst Kohlenmonoxid bereitgestellt, welches ein tatsächliches Verfahrensprodukt zumindest der in Schritt b) i) aufgeführten Methoden ist.

Für die Bereitstellung des Kohlenmonoxids ist es im Sinne der Erfindung ausreichend, wenn das Kohlenmonoxid ein Verfahrensprodukt zumindest der in Schritt b) i) aufgeführten Methoden ist. Dies bedeutet, dass es bei Durchführung des erfindungsgemäßen Verfahrens für die Ausführung des Schrittes der Bereitstellung des Kohlenmonoxids bereits ausreicht, das besagte Kohlenmonoxid als tatsächliches Verfahrensprodukt der besagten Schritte lediglich im Rahmen einer Belieferung als Rohstoff aus einem Lagerbehälter oder aus einer Zuleitung zu entnehmen. In diesem Falle führt der Hexamethylendiisocyanat-Produzent als Ausführender des erfindungsgemäßen Verfahrens die Herstellung des Kohlenmonoxids nicht selbst aus, sondern er sorgt nur dafür, dass das bereitgestellte Kohlenmonoxid durch einen Lieferanten entsprechend hergestellt wurde und ein tatsächliches Verfahrensprodukt zumindest der in Schritt b) i) aufgeführten Methoden ist.

Ebenso ist es im Rahmen einer Ausführungsform erfindungsgemäß möglich, wenn zur Bereitstellung des Kohlenmonoxids die vorgenannten Schritte zur Kohlenmonoxid Herstellung als integrale Schritte eines erfindungsgemäßen Verfahrens in Schritt b) i) durch den Hexamethylendiisocyanat-Produzenten selbst ausgeführt werden und das dabei erhaltene Kohlenmonoxid der Phosgenherstellung in Schritt b) ii) zugeführt wird.

Die in Schritt b) i) genannten Ausführungsformen der partiellen Reduktion von $CO_2$ zu CO, sowie der partiellen Oxidation von organischem Material zu CO ist, wobei mindestens ein organisches Material ausgewählt wird aus mindestens einer organischen, festförmigen Verbindung, Methan aus biologischer Quelle oder Mischungen daraus, wurden bereits unter Schritt a) i-1) beschrieben (*vide supra*). Diese gelten auch entsprechend für den Schritt b) i).

Darüber hinaus kann in Schritt b) i) das Kohlenmonoxid aus einer partiellen Oxidation von mindestens einer Monohydroxyalkylverbindung, insbesondere von Methanol, stammen. Dieser Schritt wird im Folgenden als katalytische Zersetzung bezeichnet. Dabei ist es bevorzugt, wenn die Monohydroxyalkylverbindung Methanol ist und das Methanol ein Verfahrensprodukt zumindest der unter a) i) beschriebenen Schritte ist. Hierbei sind wieder die zuvor unter Schritt a) i) beschriebenen Ausführungsformen zu berücksichtigen (*vide supra*).

Im katalytischen Zersetzungsschritt des Methanols wird bereitgestelltes Methanol im Rahmen einer bevorzugten Ausführungsform durch Inkontaktbringen von gasförmigem Methanol mit einem Katalysator und katalytisch unter Bildung von Kohlenmonoxid und gegebenenfalls zusätzlich von Wasserstoffgas zersetzt. Sofern das Methanol zu diesem Zweck nicht gasförmig vorliegt (z.B. bei Entnahme aus einem Lagerbehälter) wird dieses vorab einer Verdampfung unterworfen. Der für die Verdampfung notwendige Energieeintrag kann durch Nutzung von fossilen Brennstoffen, wie z.B. Erdgas, als Energiequelle, erzeugt werden. Für das erfindungsgemäße Verfahren wird für die Einstellung der zur Verdampfung notwendigen Temperatur besonders bevorzugt Wärmeenergie zugeführt, die mindestens durch eine Methode bereitgestellt wird, ausgewählt aus (i) der Verbrennung von Brennstoff, der mittels regenerativer Energie erzeugten Wasserstoff (gasförmiges $H_2$) enthält, (ii) der Verbrennung von Brennstoff, der Methan aus biologischer Quelle enthält oder (iii) der Umwandlung von aus regenerativer Energie erzeugter, elektrischer Energie in Wärme. Dabei ist es wiederum besonders bevorzugt, wenn als regenerative Energie wahlweise Windkraft, Sonnenenergie, Wasserkraft oder Mischungen daraus, eingesetzt wird.

Bevorzugt wird zur katalytischen Zersetzung gasförmiges Methanol über einen Katalysator, dessen Aktivkomponente mindestens eine Übergangsmetall-Spezies enthält, geleitet. Als Übergangsmetall-Spezies versteht der Fachmann alle d-Block Elemente und deren chemische Verbindungen. Besonders bevorzugt handelt es sich bei dem Katalysator um eine Übergangsmetall-Spezies, welche auf einen Träger aufgebracht ist. Ganz besonders bevorzugt handelt es sich dabei um mindestens eine Übergangsmetall-Spezies ausgewählt aus der Gruppe VII, VIII oder XI des Periodensystems der Elemente, welche wiederum bevorzugt auf einen Träger aufgebracht ist.

Die katalytische Zersetzung des Methanols wird bevorzugt bei einer Temperatur von unter 500°C, besonders bevorzugt von unter 400°C, durchgeführt. Ist für die katalytische Zersetzung von Methanol ein Energieeintrag notwendig, kann dieser durch Nutzung von fossilen Brennstoffen, wie z.B. Erdgas, als Energiequelle, bewirkt werden. Dem erfindungsgemäßen Verfahren wird für die Einstellung der zur katalytischen Zersetzung des Methanols notwendigen Temperatur besonders bevorzugt Wärmeenergie zugeführt, die mindestens durch eine Methode bereitgestellt wird, ausgewählt aus (i) der Verbrennung von Brennstoff, der mittels regenerativer Energie erzeugten Wasserstoff (gasförmiges $H_2$) enthält, (ii) der Verbrennung von Brennstoff, der Methan aus biologischer Quelle enthält oder (iii) der Umwandlung von aus regenerativer Energie erzeugter, elektrischer Energie in Wärme. Dabei ist es wiederum besonders bevorzugt, wenn als regenerative Energie wahlweise Windkraft, Sonnenenergie, Wasserkraft oder Mischungen daraus, eingesetzt wird.

Die katalytische Zersetzung des Methanols wird bevorzugt bei einer Temperatur von mindestens 200°C durchgeführt. Dabei ist es wiederum bevorzugt, wenn für die katalytische Zersetzung des Methanols die Temperatur von 200 bis von unter 500°C, besonders bevorzugt von 200 bis unter 400°C eingestellt wird.

Die katalytische Zersetzung des Methanols wird bevorzugt bei einem absoluten Druck von unter 50 bar, besonders bevorzugt von unter 40 bar, durchgeführt.

Das aus der katalytischen Zersetzung erhaltene Produktgas enthält neben Kohlenmonoxid, oftmals zusätzlich Methanol, Kohlendioxid, Wasserstoff und Wasser sowie ggf. Nebenprodukte, welche u.a. aus der Reihe der Ether (Dimethylether), Alkohole (Ethanol), Aldehyde oder Ester stammen können. Vorzugsweise wird das erhaltene Produktgas der katalytischen Zersetzung vor der Umsetzung mit Chlor zu Phosgen in einem bevorzugten, zusätzlichen Prozessschritt einem Reinigungsschritt unterzogen, in dem Methanol, Kohlendioxid, Wasserstoff und Wasser sowie ggf. Nebenprodukte vom Kohlenmonoxid abgetrennt werden.

Das für Schritt b) ii) bereitgestellte Kohlenmonoxid wird vor der Phosgenherstellung bevorzugt von

Nebenbestandteilen befreit und aufgereinigt. Zu diesem Zweck wird das Kohlenmonoxid bevorzugt einer Abtrennung von Kohlendioxid einer $CO_2$-Abtrenneinheit zugeführt, bei der das $CO_2$ abgetrennt wird.

Die $CO_2$ Abtrenneinheit und damit die Abtrennung von $CO_2$, kann als "Aminwäsche" ausgeführt werden, wobei das Kohlenmonoxid-haltige Produktgas des Reformerprozesses hier insbesondere die grundsätzlich bekannte Wäsche des Gasgemisches nach dem Prinzip der Chemisorption mit Aminen, wie Monoethanolamin (MEA) Diethanolamin (DEA), Methyldiethanolamin (MDEA) oder Diglycolamin (DGA) durchläuft, welche in einer Absorptionskolonne eine hohe Reinheit des gereinigten Gasgemisches erreicht.

In einer erfindungsgemäß weiter bevorzugten Ausführungsform des Verfahrens erfolgt eine Abtrennung von Wasser aus dem bereitgestellten Kohlenmonoxid.

Im Rahmen einer weiteren bevorzugten Ausführungsform des Verfahrens erfolgt die Abtrennung von Kohlendioxid nachdem zuvor Wasser aus dem bereitgestellten Kohlenmonoxid abgetrennt wurde. Zu diesem Zweck wird das bereitgestellte Kohlenmonoxid zunächst einer Wasser-Abtrenneinheit zugeführt, dort das Wasser abgetrennt und das nach der Abtrennung von Wasser erhaltene Kohlenmonoxid-haltige, trockene Produktgas einer $CO_2$-Abtrenneinheit zugeführt, bei der das $CO_2$ abgetrennt wird. In der Wasser-Abtrenneinheit wird zur Abtrennung des Wassers beispielsweise das bereitgestellte Kohlenmonoxid abgekühlt und das Wasser, beispielsweise als Kondensat, abgetrennt.

Als erfindungsgemäß bevorzugte Variante des Verfahrens gilt ein Verfahren, in dem das (bevorzugt vorher von Wasser und von $CO_2$ befreite) bereitgestellte Kohlenmonoxid in eine $H_2$-CO Trenneinheit eingebracht wird, in der Wasserstoff abgetrennt wird. Dabei bildet sich zumindest ein Gasstrom, dessen Gas bei 25°C und 1013 mbar mindestens 95 Vol.% Kohlenmonoxid, bevorzugter mindestens 99 Vol.% Kohlenmonoxid, enthält. Das besagte eingebrachte Kohlenmonoxid wird in der $H_2$-CO Trenneinheit vorzugsweise zunächst in zwei Gasströme aufgetrennt. Es entsteht dabei ein Gas in Form eines Gasstroms, das mindestens 95 Vol.% (bevorzugt mindestens 99 Gew.-%) Kohlenmonoxid enthält, ein weiteres Gas in Form eines Gasstroms, dessen größter Bestandteil Wasserstoff ist und unter anderem Kohlenmonoxid enthält. Das weitere Gas wird auch als Restgas der $H_2$-CO Trennung oder falls keine Restgasbehandlung erfolgt, als Endgas bezeichnet. Eine nach diesem Prinzip der Auftrennung arbeitende $H_2$-CO Trenneinheit ist die sogenannte Coldbox. Das Wasserstoff-haltige Restgas der $H_2$-CO Trennung kann wieder in die Herstellung von Methanol eingebracht werden.

Das (gegebenenfalls nach Aufreinigung) erhaltene Kohlenmonoxid wird in einem nächsten Prozessschritt b) ii), bevorzugt über einem Katalysator, zusammen mit Chlor zu Phosgen umgesetzt. Besonders bevorzugt handelt es sich bei dem Katalysator um Aktivkohle.

Für die Bereitstellung des Chlors für die im Rahmen dieser Ausführungsform erfolgende Synthese von Phosgen ist dem Fachmann die Herstellung von Chlorgas aus elektrochemischer Oxidation nach der Salzsäure-Elektrolyse mit Gasdiffusionselektrode (auch als HCl ODC Elektrolyse-Verfahren bezeichnet (ODC steht für Oxygen Depleting Electrode, als eine ODC wird beispielsweise die sogenannte SVK (Sauerstoffverzehrkathode) eingesetzt); geeignete Elektrolysezelle vgl. US,6022,634 A, WO 03/31690 A1), die Herstellung von Chlorgas aus Salzsäure-Diaphragmaelektrolyse (vgl. EP 1 103 636 A1), die Herstellung von Chlorgas aus thermokatalytischer Gasphasenoxidation (vgl. WO 2012/025483 A2), sowie die Herstellung von Chlor aus Chloralkali-Elektrolyse (vgl. WO 2009/007366 A2) hinlänglich bekannt. Auf den Inhalt der vorgenannten, im Zusammenhang mit der Herstellung von Chlorgas zitierten Schriften wird ausdrücklich und vollumfänglich Bezug genommen. Das für die Synthese von Phosgen benötigte Chlor wird in einer bevorzugten Variante dieser Ausführungsform des Verfahrens elektrolytisch hergestellt, insbesondere durch elektrochemische Oxidation nach der Salzsäure-Elektrolyse mit Gasdiffusionselektrode, durch elektrochemische Oxidation nach der Salzsäure-Diaphragmaelektrolyse oder durch elektrochemische Oxidation nach der Chloralkali-Elektrolyse. Dabei ist es wiederum besonders bevorzugt, wenn die besagte elektrochemische Oxidation jeweils unter Verwendung von aus regenerativer Energie erzeugtem elektrischem Strom durchgeführt wird, insbesondere aus regenerativer Energie in Form von Windkraft, Sonnenenergie oder Wasserkraft. In Schritt c) des erfindungsgemäßen Verfahrens wird Phosgen mit dem gemäß Schritt a) bereitgestellten Hexamethylendiamin umgesetzt und das Hexamethylendiisocyanat hergestellt. Dem Fachmann sind entsprechende Verfahren und Apparaturen zur Umsetzung von Phosgen mit organischen Aminverbindungen bekannt, beispielsweise aus der Druckschrift WO 2017/093215 A1 auf die hier ausdrücklich und vollinhaltlich Bezug genommen wird. Es hat sich im Rahmen des vorliegenden Verfahrens als vorteilhaft erwiesen, wenn in einer bevorzugten Ausführungsform das Phosgen und das Hexamethylendiamin in der Gasphase zur Umsetzung gebracht werden unter Erhalt von Hexamethylendiisocyanat. Die Phosgenierung von Aminen in der Gasphase an sich ist bekannt und kann beispielsweise erfolgen wie beschrieben in EP 0 289 840 B1, EP 1 319 655 A2, EP 1 555 258 A1, EP 1 275 639, A1, EP 1 275 640 A1, EP 1 449 826 A1, EP 1 754 698 B1, DE 10 359 627 A1, DE 10 2005 042392 A1 oder WO 2017/093215 A1.

Ein zweiter Gegenstand der Erfindung ist ein Verfahren zur Herstellung von Polyurethan, enthaltend zumindest die folgenden Schritte:

Bereitstellung von Hexamethylendiisocyanat, welches ein Verfahrensprodukt des Verfahrens des ersten Erfindungsgegenstandes ist;

Bereitstellung mindestens eines organischen Polyols;

Umsetzung des besagten Hexamethylendiisocyanats mit besagtem organischen Polyol zu Polyurethan.

Als organisches Polyol werden organische Verbindungen mit mindestens zwei Hydroxygruppen bezeichnet. Diese werden bevorzugt ausgewählt aus Polyesterpolyol, Polyetherpolyol, Polycarbonatpolyol, Polyetheresterpolyol, Polyacrylatpolyol Polyesterpolyacrylatpolyol oder Mischungen daraus, besonders bevorzugt ausgewählt aus der Gruppe der Polyetherpolyole und/oder der Polyesterpolyole.

Im Rahmen einer bevorzugten Ausführungsform, kennzeichnet sich das Verfahren zur Herstellung von Polyurethan dadurch, dass das die Bereitstellung des Hexamethylendiisocyanats unter Anwendung eines Mehrkomponentensystems nach drittem Gegenstand der Erfindung (*vide infra*) erfolgt.

Ein dritter Gegenstand der Erfindung ist ein Mehrkomponenten-System zur Hexamethylendiisocyanat Herstellung nach einem Herstellungsverfahren des ersten Erfindungsgegenstandes, enthaltend

i) ein Volumen befüllt mit Hexamethylendiamin, welches ein Verfahrensprodukt des Schrittes a) des Verfahrens gemäß erstem Erfindungsgegenstand ist;

ii) ein Volumen befüllt mit Kohlenmonoxid, welches ein Verfahrensprodukt des Schrittes b) i-1) des Verfahrens gemäß erstem Erfindungsgegenstand ist;

iii) mindestens eine Elektrolysevorrichtung zur Herstellung von Chlor;

iv) mindestens einen Reaktor zur Herstellung von Phosgen, wobei der Reaktor mindestens einen Einlass für Chlor aufweist, der in Fluidverbindung mit der Elektrolysevorrichtung zur Herstellung von Chlor steht, und mindestens einen Einlass für Kohlenmonoxid aufweist, der in Fluidverbindung mit dem Volumen befüllt mit dem Kohlenmonoxid steht;

v) mindestens ein Reaktor zur Herstellung von Hexamethylendiisocyanat, welcher mindestens einen Einlass für besagtes Phosgen und mindestens einen Einlass für besagtes Hexamethylendiamin enthält, wobei der Einlass für besagtes Phosgen in Fluidverbindung mit dem Reaktor zur Herstellung von Phosgen und der Einlass für besagtes Hexamethylendiamin in Fluidverbindung mit dem Volumen des besagten Hexamethylendiamins steht.

Alle Ausführungsformen des Schritts a) des erfindungsgemäßen Verfahrens zur Herstellung von Hexamethylendiisocyanat sind auch für das Mehrkomponenten-System mutatis mutandis gültig.

Alle Ausführungsformen des Schritts b) des erfindungsgemäßen Verfahrens zur Herstellung von Hexamethylendiisocyanat sind auch für das Mehrkomponenten-System mutatis mutandis gültig.

Ein vierter Gegenstand der Erfindung ist daher die Verwendung von Methanol als Rohstoff zur Herstellung des Hexamethylen-Rests von Hexamethylendiisocyanat. Dabei ist eine entsprechende Verwendung bevorzugt, die nach einem Verfahren gemäß erstem Erfindungsgegenstand erfolgt.

## Patentansprüche

**1.** Verfahren zur Herstellung von Hexamethylendiisocyanat für die Herstellung von Polyurethan, enthaltend zumindest die Schritte:

a) Bereitstellung von Hexamethylendiamin, welches ein Verfahrensprodukt eines Verfahrens enthaltend mindestens folgende Schritte ist:

i) Bereitstellung von Methanol, welches ein Verfahrensprodukt eines Verfahrens mit mindestens folgenden Schritten ist:

i-1) Bereitstellung von Kohlenmonoxid als Verfahrensprodukt mindestens einer partiellen Reduktion von $CO_2$ zu CO und/oder als Verfahrensprodukt mindestens einer partiellen Oxidation von organischem Material zu CO ist, wobei mindestens ein organisches Material ausgewählt wird aus mindestens einer organischen, festförmigen Verbindung, Methan aus biologischer Quelle oder Mischungen daraus;
i-2) Umsetzung des bereitgestellten Kohlenmonoxids zu Methanol;

ii) Synthese von Propen aus zuvor bereitgestelltem Methanol durch zumindest folgende Schritte:

ii-1) Umsetzung des zuvor bereitgestellten Methanols zu einer Produktmischung, enthaltend Dimethylether, Wasser und Methanol;
ii-2) Umwandlung von Ausgangsmaterial, enthaltend Dimethylether, Wasser und Methanol jeweils aus der vorgenannten Produktmischung, bei einer Temperatur von mehr als 200°C durch Kontakt mit einem Katalysator, vorzugsweise mit mindestens einer Zeolith-Verbindung als Katalysator, zu Propen;

iii) Synthese von Acrylnitril aus dem Propen durch zumindest folgende Schritte:

iii-1) Bereitstellung von Ammoniak, welcher ein Verfahrensprodukt eines Verfahrens mit mindestens folgenden Schritten ist:

iii-1-1) Bereitstellung von Wasserstoffgas als Produkt einer Elektrolyse von Wasser, bevorzugt unter Einsatz von aus regenerativer Energie erzeugter elektrischer Energie;
iii-1-2) Umsetzung des bereitgestellten Wasserstoffgases mit gasförmigem Stickstoff zu Ammoniak;

iii-2) Umsetzung des Propens mit dem bereitgestellten Ammoniak zu Acrylnitril;

iv) Synthese von Adiponitril aus dem Acrylnitril durch zumindest folgende Schritte:

Einbringung einer Mischung, enthaltend Wasser, besagtes Acrylnitril und mindestens ein Elektrolytsalz in den Kathodenraum einer Elektrolysezelle und Inkontaktbringen der Mischung mit einer Kathode, an der elektrischer Strom anliegt;
kathodische Hydrodimerisierung des Acrylnitrils unter Bildung einer Adiponitril-haltigen Produktmischung;
Ausbringung der Adiponitril-haltigen Produktmischung aus dem Kathodenraum der Elektrolysezelle und optional Aufreinigung des Adiponitrils;

v) Synthese von Hexamethylendiamin aus dem Adiponitril durch zumindest folgende Schritte:

Bereitstellung von Wasserstoffgas als Produkt einer Elektrolyse, bevorzugt unter Einsatz von aus regenerativer Energie erzeugter elektrischer Energie;
Hydrierung des Adiponitrils mit dem Wasserstoffgas zu Hexamethylendiamin;

b) Herstellung von Phosgen durch zumindest folgende Verfahrensschritte:

i) Bereitstellung von Kohlenmonoxid als Verfahrensprodukt mindestens einer partiellen Reduktion von $CO_2$ zu CO und/oder als Verfahrensprodukt mindestens einer partiellen Oxidation von organischem Material zu CO, wobei mindestens ein organisches Material ausgewählt wird aus mindestens einer organischen, festförmigen Verbindung, einer organischen, flüssigen Monohydroxyalkylverbindung, Methan aus biologischer Quelle oder Mischungen daraus;
ii) Umsetzung des Kohlenmonoxids aus Schritt i) mit Chlor zu Phosgen;

c) Umsetzung des bereitgestellten Hexamethylendiamins mit dem Phosgen zu Hexamethylendiisocyanat.

**2.** Verfahren gemäß Anspruch 1, **dadurch gekennzeichnet, dass** das in Schritt a) i-1) und/oder das in Schritt b) i-1) bereitgestellte Kohlenmonoxid jeweils unabhängig ein Verfahrensprodukt mindestens einer partiellen Reduktion eines Gasstroms ist, der mindestens $CO_x$ mit x = 1 oder 2 und gegebenenfalls Wasserstoffgas enthält.

**3.** Verfahren gemäß einem der Ansprüche 1 oder 2, **dadurch gekennzeichnet, dass** das in Schritt a) i-1) und/oder das in Schritt b) i-1) bereitgestellte Kohlenmonoxid jeweils unabhängig ein Verfahrensprodukt mindestens einer partiellen Reduktion von $CO_2$ zu CO ist, insbesondere ausgewählt aus einer reverse water-gas-shift Reaktion unter Einsatz von mittels Elektrolyse bereitgestelltem $H_2$, aus einer elektrochemischen Reduktion von $CO_2$ zu CO oder aus Mischungen daraus.

**4.** Verfahren gemäß einem der vorangehenden Ansprüche, **dadurch gekennzeichnet, dass** das in Schritt a) i-1) und/oder das in Schritt b) i-1) bereitgestellte Kohlenmonoxid jeweils unabhängig als Verfahrensprodukt mindestens einer reverse water-gas-shift Reaktion unter Einsatz von mittels Elektrolyse bereitgestelltem $H_2$ ist oder aus einer elektrochemischen Reduktion von $CO_2$ zu CO oder aus Mischungen daraus bereitgestellt wird, mit der Maßgabe, dass hierbei jeweils aus regenerativer Energie hergestellte elektrischer Energie genutzt wird.

**5.** Verfahren gemäß einem der vorangehenden Ansprüche, **dadurch gekennzeichnet, dass** die Umsetzung des Methanols gemäß Schritt a) ii-1) durch Kontaktieren des Methanols, bevorzugt des Methanols in der Gasphase, mit einem Katalysator erfolgt.

**6.** Verfahren gemäß einem der vorangehenden Ansprüche, **dadurch gekennzeichnet, dass** zur Umsetzung des Methanols in Schritt a) ii-1) das besagte bereitgestellte Methanol eine Temperatur in einem

Bereich von 200 °C bis 350°C aufweist.

**7.** Verfahren gemäß einem der vorangehenden Ansprüche, **dadurch gekennzeichnet, dass** die Umwandlung des Ausgangsmaterials gemäß Schritt a) ii-2) bei einer Temperatur von 350°C bis 600°C, insbesondere von 380°C bis 550°C, erfolgt.

**8.** Verfahren gemäß einem der vorangehenden Ansprüche, **dadurch gekennzeichnet, dass** die Umsetzung des bereitgestellten Ammoniaks mit dem Propen gemäß Schritt a) iii-2) unter Einsatz von Luftsauerstoff bei einer Temperatur von 350°C bis 550°C erfolgt.

**9.** Verfahren gemäß einem der vorangehenden Ansprüche, **dadurch gekennzeichnet, dass** die Umsetzung des bereitgestellten Ammoniaks mit dem Propen gemäß Schritt a) iii-2) an einem Molybdat- und/oder Antimonat-basierten Katalysator erfolgt.

**10.** Verfahren gemäß einem der vorangehenden Ansprüche, **dadurch gekennzeichnet, dass** das Elektrolytsalz im Kathodenraum der Elektrolysezelle ausgewählt wird aus mindestens einem quartären Ammoniumsalz, insbesondere mindestens einem quartären Ammoniumsulfonat.

**11.** Verfahren gemäß einem der vorangehenden Ansprüche, **dadurch gekennzeichnet, dass** in Schritt a) iv) die Elektrolysezelle einen Kathodenraum und einen Anodenraum aufweist, die durch eine Membran voneinander getrennt sind.

**12.** Verfahren gemäß einem der vorangehenden Ansprüche **dadurch gekennzeichnet, dass** in Schritt a) iv) die eingebrachte Mischung einen pH-Wert im Bereich von pH 7 bis pH 10, insbesondere von pH 7 bis pH 9,5.

**13.** Verfahren gemäß einem der vorangehenden Ansprüche, **dadurch gekennzeichnet, dass** an der Elektrolysezelle ein elektrischer Strom mit einer Stromdichte von 15 bis40 A/dm$^2$ anliegt.

**14.** Verfahren zur Herstellung von Polyurethan, enthaltend zumindest die folgenden Schritte:

Bereitstellung von Hexamethylendiisocyanat, welches ein Verfahrensprodukt des Verfahrens nach einem der Ansprüche 1 bis 13 ist;
Bereitstellung mindestens eines organischen Polyols;
Umsetzung des besagten Hexamethylendiisocyanat mit besagtem Polyol zu Polyurethan.

**15.** Verfahren nach Anspruch 14, **dadurch gekennzeichnet, dass** das die Bereitstellung des Hexamethylendiisocyanats unter Anwendung eines Mehrkomponentensystems nach Anspruch 16 erfolgt.

**16.** Mehrkomponenten-System zur Hexamethylendiisocyanat Herstellung nach einem Herstellungsverfahren der Ansprüche 1 bis 13, enthaltend

i) ein Volumen befüllt mit Hexamethylendiamin, welches ein Verfahrensprodukt des Schrittes a) des Verfahrens gemäß einem der Ansprüche 1 bis 13 ist;
ii) ein Volumen befüllt mit Kohlenmonoxid, welches ein Verfahrensprodukt des Schrittes b) i-1) des Verfahrens gemäß einem der Ansprüche 1 bis 13 ist;
iii) mindestens eine Elektrolysevorrichtung zur Herstellung von Chlor;
iv) mindestens einen Reaktor zur Herstellung von Phosgen, wobei der Reaktor mindestens einen Einlass für Chlor aufweist, der in Fluidverbindung mit der Elektrolysevorrichtung zur Herstellung von Chlor steht, und mindestens einen Einlass für Kohlenmonoxid aufweist, der in Fluidverbindung mit dem Volumen befüllt mit dem Kohlenmonoxid steht;
v) mindestens ein Reaktor zur Herstellung von Hexamethylendiisocyanat, welcher mindestens einen Einlass für besagtes Phosgen und mindestens einen Einlass für besagtes Hexamethylendiamin enthält, wobei der Einlass für besagtes Phosgen in Fluidverbindung mit dem Reaktor zur Herstellung von Phosgen und der Einlass für besagtes Hexamethylendiamin in Fluidverbindung mit dem Volumen des besagten Hexamethylendiamins steht.

**17.** Verwendung von Methanol als Rohstoff zur Herstellung des Hexamethylen-Rests von Hexamethylendiisocyanat, bevorzugt nach einem Verfahren gemäß einem der Ansprüche 1 bis 13.

Europäisches Patentamt
European Patent Office
Office européen des brevets

# EUROPÄISCHER RECHERCHENBERICHT

Nummer der Anmeldung
EP 23 16 6923

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2021/089737 A1 (COVESTRO INTELLECTUAL PROPERTY GMBH & CO KG [DE]) 14. Mai 2021 (2021-05-14) * das ganze Dokument * | 1-13,16 | INV. C25B1/04 C25B1/23 C25B1/26 C25B3/09 |
| X,D | WO 2021/069470 A1 (COVESTRO INTELLECTUAL PROPERTY GMBH & CO KG [DE]) 15. April 2021 (2021-04-15) * das ganze Dokument * | 1-13,16 | C25B3/29 C25B15/08 C01B3/12 C01B32/40 C01B32/80 |
| X | WO 2020/239716 A1 (COVESTRO INTELLECTUAL PROPERTY GMBH & CO KG [DE]) 3. Dezember 2020 (2020-12-03) * das ganze Dokument * | 1-13,16 | C01C1/04 C07C1/20 C07C29/151 C07C209/48 C07C253/26 |
| A | CN 113 480 448 A (BEIJING RISUNTECHNOLOGY CO LTD) 8. Oktober 2021 (2021-10-08) * Einleitung * | 1-13,16 | C07C263/10 C08G18/73 |
| A | DE 10 2020 200905 A1 (THYSSENKRUPP AG [DE]; THYSSENKRUPP IND SOLUTIONS AG [DE]) 29. Juli 2021 (2021-07-29) * Ansprüche 1, 4 * | 1-13,16 | |

RECHERCHIERTE SACHGEBIETE (IPC)

C25B
C01B
C01C
C07C
C08G

Der vorliegende Recherchenbericht wurde für alle Patentansprüche erstellt

1

| Recherchenort | Abschlußdatum der Recherche | Prüfer |
|---|---|---|
| München | 23. Oktober 2023 | Ritter, Thomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

Nummer der Anmeldung

EP 23 16 6923

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

**Siehe Ergänzungsblatt B**

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

**1-13, 16**

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches Patentamt
European Patent Office
Office européen des brevets

# MANGELNDE EINHEITLICHKEIT DER ERFINDUNG ERGÄNZUNGSBLATT B

Nummer der Anmeldung
EP 23 16 6923

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

**1. Ansprüche: 1-13, 16**

**Verfahren zur Herstellung von Hexamethylendiisocyanat enthaltend die Schritte a) bis c) wie in Anspruch 1. Entsprechendes Mehrkomponenten-System (Anspruch 16).**

---

**2. Ansprüche: 14, 15**

**Verfahren zur Herstellung von Polyurethan enthaltend die Schritte des Anspruchs 14.**

---

**3. Anspruch: 17**

**Verwendung von Methanol als Rohstoff zur Herstellung des Hexamethylen-Rests von Hexamethylendiisocyanat.**

---

## ANHANG ZUM EUROPÄISCHEN RECHERCHENBERICHT ÜBER DIE EUROPÄISCHE PATENTANMELDUNG NR.

EP 23 16 6923

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-10-2023

| Im Recherchenbericht angeführtes Patentdokument | Datum der Veröffentlichung | Mitglied(er) der Patentfamilie | Datum der Veröffentlichung |
|---|---|---|---|
| WO 2021089737 A1 | 14-05-2021 | CN 114599635 A | 07-06-2022 |
| | | EP 3819259 A1 | 12-05-2021 |
| | | EP 4054975 A1 | 14-09-2022 |
| | | JP 2023500262 A | 05-01-2023 |
| | | KR 20220098137 A | 11-07-2022 |
| | | US 2022389150 A1 | 08-12-2022 |
| | | WO 2021089737 A1 | 14-05-2021 |
| WO 2021069470 A1 | 15-04-2021 | CN 114450436 A | 06-05-2022 |
| | | EP 3805429 A1 | 14-04-2021 |
| | | EP 4041939 A1 | 17-08-2022 |
| | | JP 2022551135 A | 07-12-2022 |
| | | KR 20220079553 A | 13-06-2022 |
| | | WO 2021069470 A1 | 15-04-2021 |
| WO 2020239716 A1 | 03-12-2020 | CN 113853398 A | 28-12-2021 |
| | | EP 3744812 A1 | 02-12-2020 |
| | | ES 2909402 T3 | 06-05-2022 |
| | | HU E057982 T2 | 28-06-2022 |
| | | JP 2022534099 A | 27-07-2022 |
| | | KR 20220012858 A | 04-02-2022 |
| | | PT 3744812 T | 24-03-2022 |
| | | US 2022227701 A1 | 21-07-2022 |
| | | WO 2020239716 A1 | 03-12-2020 |
| CN 113480448 A | 08-10-2021 | KEINE | |
| DE 102020200905 A1 | 29-07-2021 | DE 102020200905 A1 | 29-07-2021 |
| | | EP 4097051 A1 | 07-12-2022 |
| | | US 2023034962 A1 | 02-02-2023 |
| | | WO 2021151672 A1 | 05-08-2021 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2022052267 W
- WO 2021089737 A
- WO 2021069470 A
- WO 2020057998 A1
- EP 0448000 B1
- DE 19723363 A1
- US 7015369 B2
- US 2904580 A
- US 3193480 A
- US 3232888 A
- US 3821305 A
- US 3773832 A
- US 4598058 A
- US 3696153 A
- US 4587228 A
- US 6022634 A
- WO 0331690 A1
- EP 1103636 A1
- WO 2012025483 A2
- WO 2009007366 A2
- WO 2017093215 A1
- EP 0289840 B1
- EP 1319655 A2
- EP 1555258 A1
- EP 1275639 A1
- EP 1275640 A1
- EP 1449826 A1
- EP 1754698 B1
- DE 10359627 A1
- DE 102005042392 A1


### In der Beschreibung aufgeführte Nicht-Patentliteratur


- **DITTMEYER et al.** Chemische Technik. vol. 4
- **VOLKMAR M. SCHMIDT.** Elektrochemische Verfahrenstechnik. Wiley-VCH-Verlag, 2003
- **JÜRGEN CARL EF-VERL.** Noell-Konversionsverfahren zur Verwertung und Entsorgung von Abfällen. *Energie- und Umwelttechnik,* 1994, ISBN 3924511829
- **OTT et al.** Methanol in: Ullmann’s Encyclopedia of industrial chemistry. Wiley-VCH Verlag
- *CHEMICAL ABSTRACTS,* 111-69-3